# EUROPEAN PATENT APPLICATION

(11) **EP 4 361 268 A1**
(43) Date of publication of application: **01.05.2024**
(21) Application number: 22819356.1
(22) Date of filing: 24.05.2022
(51) Int. Cl.: C12N 15/113, C12N 15/09, C12N 1/20, C07K 14/34, C12N 15/67, C12R 1/15, C12R 1/185, C12R 1/13, C12R 1/06

(54) **MDH GENE-BASED POLYNUCLEOTIDE HAVING PROMOTER ACTIVITY AND USE THEREOF**

(30) Priority: 08.06.2021 CN 202110638496
(71) Applicant: Tianjin Institute of Industrial Biotechnology, Chinese Academy of Sciences, Tianjin Airport Economic Area Tianjin 300308 (CN)
(72) Inventor: ZHENG, Ping, Tianjin 300308 (CN); LIU, Jiao, Tianjin 300308 (CN); SUN, Jibin, Tianjin 300308 (CN); SUN, Guannan, Tianjin 300308 (CN); ZHOU, Wenjuan, Tianjin 300308 (CN); GUO, Xuan, Tianjin 300308 (CN)
(74) Representative: Weickmann & Weickmann PartmbB
(86) International application number: PCT/CN2022/094736
(87) International publication number: WO 2022/257758

(57) **Abstract**

A polynucleotide having promoter activity of a malate dehydrogenase gene (mdh gene), a transcription expression cassette, recombinant expression vector and recombinant host cell containing the polynucleotide having the promoter activity, a method for constructing a promoter mutant, a method for regulating the transcription of a target gene, a method for preparing a protein, and method for producing a target compound. The polynucleotide having the promoter activity is a mutant of an mdh gene promoter, and compared with a promoter of a wild-type mdh gene, the promoter activity of the mutant is significantly improved. After operably linking the mutant to a target gene, the expression efficiency of the target gene can be significantly improved, thereby effectively improving the yield and transformation rate of a target compound.

## Description

### TECHNICAL FIELD

The present disclosure pertains to the field of biotechnology and genetic engineering, and particularly relates to a polynucleotide having promoter activity, and a transcription expression cassette, a recombinant expression vector and a recombinant host cell comprising the polynucleotide having promoter activity, as well as a method for constructing a promoter mutant, a method for regulating transcription of a target gene, and a method for preparing a protein and a method for producing a target compound.

### BACKGROUND

Microbial fermentation can produce a variety of target compounds, such as amino acids and organic acids, which can be widely used in medicine, food, animal feed and cosmetics, and have great economic value. In recent years, with the increasing market demand for amino acids, organic acids and so on, how to increase the output of a target compound and realize industrial large-scale production of the target compound is an important problem to be solved urgently.

Breeding high-yield fermentation microorganisms is an important means to improve the industrial yield of target compounds. Compared with traditional mutation breeding technology, genetic engineering breeding technology has been widely used because of its strong pertinence and high efficiency. Numerous studies have shown that the efficient expression of a key gene in the synthesis pathway of a target compound is the key to improve the yield and conversion rate of the target compound.

It is an important method to improve the fermentation yield of a target compound by transforming a key gene in the microbial metabolic pathway through genetic engineering. Promoter is an important regulatory element that affects gene expression, and fine regulation of the promoter can optimize the conversion rate of the target compound. Promoters with different expression intensities can meet the needs of different expression intensities of different genes, thereby improving the yield and conversion rate of the target compound.

Therefore, it is an important problem to be solved urgently in the field of microbial fermentation to develop more promoters with high activity so as to enhance the expression of key genes in the synthesis pathway of target compounds, improve the yield of target compounds and enhance the competitiveness of bio-fermentation industry.

### SUMMARY

### Technical problem

In view of the technical problem existing in the prior art, for example, it is necessary to develop more promoters with high activity so as to enhance the expression of key genes in the synthesis pathway of target compounds, the present disclosure provides a polynucleotide having promoter activity, which is a mutant of the polynucleotide comprising the sequence as set forth in SEQ ID NO: 1. The mutant provided by the present disclosure has promoter activity significantly improved as compared with the wild-type promoter, and provides an expression regulatory element with great application potential for the transformation of a target gene. By operably ligating the mutant with the target gene, the expression of the target gene can be effectively improved, thereby effectively improving the yield and conversion rate of a target compound.

### Solution to problem

The present disclosure provides a polynucleotide having promoter activity, wherein the polynucleotide is selected from the group shown in any one of the following (i)-(iv):
(i) a mutant of a polynucleotide comprising the sequence as set forth in SEQ ID NO: 1, comprising a mutated nucleotide at one or more positions in position 105 to position 126 of the sequence as set forth in SEQ ID NO: 1;
(ii) a polynucleotide comprising a sequence complementary to the nucleotide sequence as set forth in (i);
(iii) a polynucleotide comprising a reversely complementary sequence of a sequence capable of hybridizing with the nucleotide sequence as set forth in (i) or (ii) under high stringent hybridization conditions or very high stringent hybridization conditions;
(iv) a polynucleotide having at least 90%, optionally at least 95%, preferably at least 97%, more preferably at least 98%, and most preferably at least 99% sequence identity compared with the nucleotide sequence as set forth in (i) or (ii);
wherein the polynucleotide as set forth in any one of (i) to (iv) has a nucleotide sequence that is not TTCTCTTTTAAGCGGGATAGCA at position 105 to position 126 of the sequence as set forth in SEQ ID NO: 1; and the polynucleotide as set forth in any one of (i) to (iv) has enhanced promoter activity compared with the polynucleotide having the sequence as set forth in SEQ ID NO: 1.

In some embodiments, according to the polynucleotide having promoter activity of the present disclosure, wherein the mutant has 0.4 to 13 folds or more improved promoter activity as compared with the promoter activity of the polynucleotide having the sequence as set forth in SEQ ID NO: 1.

In some embodiments, according to the polynucleotide having promoter activity of the present disclosure, wherein the nucleotide sequence of the mutant corresponding to position 105 to position 126 of the sequence as set forth in SEQ ID NO: 1 is selected from any one of the group consisting of the following (P*_{mdh}*-1) to (P*_{mdh}*-49):
(P*_{mdh}*-1) CAGGTAACAACTGCGAGTACGG;
(P*_{mdh}*-2) AGTCTGAGGGATATAATTAAGA;
(P*_{mdh}-*3) GGTCGTGGAGGACAAGTTAAGA;
(P*_{mdh}*-4) CTACTACCATCGGGTGCTAAGG;
*(*P*_{mdh}*-5) GATAGGTAGGCGTTAAGTAAAC;
(P*_{mdh}*-6) GGCAATTCGCGGGGAGGTAGAC;
(P*_{mdh}-*7) TATTGGGCGCGGCATTATAGGA;
(P*_{mdh}*-8) GGGAACTGCTTTGGTAGTATCA;
(P*_{mdh}*-9) GTTCAGGTTTGGGCCGCTATGC;
(P*_{mdh}*-10) TGA TCGGGGCGCTTTGGT ACGG;
(P*_{mdh}*-11) TATGAAATAAATTGGAGTAGGC;
(P*_{mdh}*-12) AACGGTATCTTTCTCCATATAA;
(P*_{mdh}*-13) AACGGAGCGTGTACGACTAGAA;
(P*_{mdh}*-14) TGCGCGGGTAAACGCTGTAGAG;
(P*_{mdh}*-15) TTGATGTCGTAGCGTAGTATTA;
(P*_{mdh}-*16) AGCTCGGACTTTGGTGGTAGGC;
(P*_{mdh}*-17) CCGGCGCATTATAACGCTACAA;
(P*_{mdh}*-18) CGGGGGTGTAAGTCGAGTAGAC;
(P*_{mdh}*-19) ACGGGTAGGTATTTCGCTAGGA;
(P*_{mdh}*-20) TAGTGTATGCTTTTACATAGAC;
(P*_{mdh}-*21) AGGCAGCATGTACATGATATCG;
(P*_{mdh}*-22) TTGAGCGGAGTTAATAGTACAC;
*(*P*_{mdh}*-23) ATACACACAACTTTCGATACTA;
(P*_{mdh}*-24) ATTCCAACCTTATGTTATACTG;
(P*_{mdh}*-25) GAGGTAGTCGTTTGGGGTAGGA;
(P*_{mdh}*-26) ATTGCTCGCGTAAATGGTAAAC;
(P*_{mdh}*-27) TGTTATTATGGGAGATGTAAGT;
(P*_{mdh}*-28) GAAAAATGTACGTGTGTTAAAA;
(P*_{mdh}*-29) AGTCCTCAGTGGAGTGGTAACA;
(P*_{mdh}*-30) TTTCCGGTGTTTTCGCGTAGAG;
(P*_{mdh}*-31) GGCGTCGGTAGGTATGTTATTG;
(P*_{mdh}*-32) ACGGCGATCAGAGTGTGTAAGA;
(P*_{mdh}*-33) CCGTGCGTGATATGGGGTAGTG;
(P*_{mdh}*-34) GGGTTGTGAGTTATCGGTATAA;
(P*_{mdh}*-35) TGCTCCGATGATGGTGGTAAGA;
(P*_{mdh}*-36) GTTCTCACGGGTCCGTGTATGA;
(P*_{mdh}*-37) TTTCGGAATGGATGGTGTACGC;
(P*_{mdh}*-38) ATTATGTCTTCTTTTGGTAAAA;
(P*_{mdh}*-39) GATCATAGGTTCTGTGGTAGAT;
(P*_{mdh}*-40) CATGGCCGATCATGATGTAATA;
(P*_{mdh}*-41) TAGTGGCGGTGTACGTGTATGA;
(P*_{mdh}*-42) ATTTGTTACAGGTATGGTAGTT;
(P*_{mdh}*-43) ACGGAACTTATTTCTGGTAACG;
(P*_{mdh}*-44) ACCGGGAGGACTAATGGTATGG;
(P*_{mdh}*-45) GCCACCCCTTTTTCTGCTATTA;
(P*_{mdh}*-46) TTGCGCCGGAGTTGCGGTATAG;
(P*_{mdh}*-47) GTTTACGGGTGTTCATGTAGTA;
(P*_{mdh}*-48) TTTGTCTCGTATCTGTGTAAGA;
(P*_{mdh}*-49) ATTTTGGGCACTTAGTGTATTG.

In some embodiments, according to the polynucleotide having promoter activity of the present disclosure, wherein the nucleotide sequence of the mutant is selected from sequences as set forth in any one of SEQ ID NOs: 3 to 51.

The present disclosure further provides a transcription expression cassette, comprising the polynucleotide having promoter activity according to the present disclosure; optionally, the transcription expression cassette further contains a target gene that is operably ligated to the polynucleotide having promoter activity; preferably, the target gene is a protein coding gene.

The present disclosure further provides a recombinant expression vector comprising the polynucleotide having promoter activity according to the present disclosure, or the transcription expression cassette of the present disclosure.

The present disclosure further provides a recombinant host cell comprising the transcription expression cassette of the present disclosure, or the recombinant expression vector of the present disclosure.

In some embodiments, according to the recombinant host cell of the present disclosure, wherein the host cell is from genus *Corynebacterium,* genus *Brevibacterium,* genus *Arthrobacterium,* genus *Microbacterium* or genus *Escherichia*; preferably, the host cell is *Corynebacterium glutamicum* or *Escherichia coli*; more preferably, the host cell is *Corynebacterium glutamicum* ATCC 13032, *Corynebacterium glutamicum* ATCC 13869, *Corynebacterium glutamicum* ATCC 14067 or derivative strains of *Corynebacterium glutamicum.*

The present disclosure further provides the use of the polynucleotide having promoter activity according to the present disclosure, the transcription expression cassette according to the present disclosure, the recombinant expression vector according to the present disclosure, and the recombinant host cell according to the present disclosure in at least one of the following:
a) enhancing the transcription level of a gene, or preparing a reagent or kit for enhancing the transcription level of a gene;
b) preparing a protein, or preparing a reagent or kit for preparing a protein;
c) producing a target compound, or preparing a reagent or kit for producing a target compound.

In some embodiments, according to the use of the present disclosure, wherein the protein is selected from a gene expression regulatory protein, a protein related to the synthesis of a target compound, or a protein related to membrane transport.

In some embodiments, according to the use of the present disclosure, wherein the target compound includes at least one of amino acids, organic acids;
optionally, the amino acids include one or a combination of two or more of the following: proline, hydroxyproline, lysine, glutamic acid, threonine, glycine, alanine, valine, leucine, isoleucine, serine, cysteine, glutamine, methionine, aspartic acid, asparagine, arginine, histidine, phenylalanine, tyrosine, tryptophan, 5-aminolevulinic acid or derivatives of any one of the above amino acids;
optionally, the organic acids include one or a combination of two or more of the following: citric acid, succinic acid, lactic acid, acetic acid, butyric acid, palmitic acid, oxalic acid, oxaloacetic acid, tartaric acid, propionic acid, hexenoic acid, decanoic acid, caprylic acid, valeric acid, malic acid or derivatives of any one of the above amino acids.

The present disclosure further provides a construction method of a promoter mutant, including the following steps:

Mutation step: mutating a polynucleotide having the sequence as set forth in SEQ ID NO: 1 so that there are mutated nucleotides at one or more positions in position 105 to position 126 of the sequence as set forth in SEQ ID NO: 1;

Screening step: screening for a mutant of a polynucleotide having improved promoter activity as compared with the polynucleotide having the sequence as set forth in SEQ ID NO: 1, to obtain a promoter mutant.

In some embodiments, according to the construction method of the present disclosure, wherein the mutation step includes:

mutating the polynucleotide having the sequence as set forth in SEQ ID NO: 1 so that the nucleotides at position 105 to position 126 of the sequence as set forth in SEQ ID NO: 1 are mutated to a nucleotide sequence as set forth below: NNNNNNNNNNNNNNNNNTANNNT; wherein N is selected from A, T, C or G;

preferably, the promoter mutant has 0.4 to 13 folds or more improved promoter activity as compared with the promoter activity of the polynucleotide having the sequence as set forth in SEQ ID NO: 1.

The present disclosure further provides a method for regulating transcription, wherein the method includes a step of operably ligating the polynucleotide having promoter activity of the present disclosure to a target RNA or a target gene. Optionally, the target RNA includes at least one of tRNA, sRNA, the target gene includes at least one of a coding gene of a protein related to the synthesis of a target compound, a coding gene of a gene expression regulatory protein and a coding gene of a protein related to membrane transport;
optionally, the target gene includes at least one of the following genes: pyruvate carboxylase gene, phosphoenolpyruvate carboxylase gene, γ-glutamyl kinase gene, glutamate semialdehyde dehydrogenase gene, pyrroline-5-carboxylate reductase gene, amino acid transport protein gene, ptsG system related gene, pyruvate dehydrogenase gene, homoserine dehydrogenase gene, oxaloacetic acid decarboxylase gene, gluconic acid repressor gene, glucose dehydrogenase gene, aspartate kinase gene, aspartate semialdehyde dehydrogenase gene, aspartate ammonia lyase gene, dihydrodipicolinate synthase gene, dihydropyridinic acid reductase gene, succinyl diaminopimelate aminotransferase gene, tetrahydrodipicolinate succinylase gene, succinyl diaminopimelate deacylase gene, diaminopimelic acid epimerase gene, diaminopimelic acid deacylase gene, glyceraldehyde-3-phosphate dehydrogenase gene, transketolase gene, diaminopimelate dehydrogenase gene.

The present disclosure further provides a method for preparing a protein, wherein the method includes a step of expressing the protein by using the transcription expression cassette of the present disclosure, the recombinant expression vector of the present disclosure, or the recombinant host cell of the present disclosure; optionally, the protein is a protein related to the synthesis of a target compound, a protein related to membrane transport, or a gene expression regulatory protein;
optionally, the method further includes a step of isolating or purifying the protein.

The present disclosure further provides a method for producing a target compound, wherein the method includes a step of expressing a protein related to the synthesis of a target compound, a protein related to membrane transport, or a gene expression regulatory protein by using the transcription expression cassette of the present disclosure, the recombinant expression vector of the present disclosure, or the recombinant host cell of the present disclosure, and producing the target compound in the presence of the protein associated with the synthesis of the target compound or the gene expression regulatory protein;
optionally, the target compound includes at least one of amino acids, organic acids;
optionally, the amino acids include one or a combination of two or more of the following: lysine, glutamic acid, threonine, proline, hydroxyproline, glycine, alanine, valine, leucine, isoleucine, serine, cysteine, glutamine, methionine, aspartic acid, asparagine, arginine, histidine, phenylalanine, tyrosine, tryptophan, 5-aminolevulinic acid or derivatives of any one of the above amino acids;
optionally, the organic acids include one or a combination of two or more of the following: citric acid, succinic acid, lactic acid, acetic acid, butyric acid, palmitic acid, oxalic acid, oxaloacetic acid, tartaric acid, propionic acid, hexenoic acid, decanoic acid, caprylic acid, valeric acid, malic acid or derivatives of any one of the above organic acids;
optionally, the protein related to the synthesis of the target compound is a protein related to the synthesis of an L-amino acid; optionally, the protein related to the synthesis of an L-amino acid includes one or a combination of two or more of: pyruvate carboxylase, phosphoenolpyruvate carboxylase, γ-glutamyl kinase, glutamate semialdehyde dehydrogenase, pyrroline-5-carboxylate reductase, amino acid transport protein, ptsG system, pyruvate dehydrogenase, homoserine dehydrogenase, oxaloacetic acid decarboxylase, gluconic acid repressor, glucose dehydrogenase, aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, transketolase, diaminopimelate dehydrogenase.
optionally, the method further includes a step of isolating or purifying the target compound.

### Effects

In some embodiments, the polynucleotide having promoter activity provided by the present disclosure is a mutant of malate dehydrogenase gene (*mdh* gene) promoter, and the mutant has a promoter activity significantly improved as compared with the wild-type *mdh* gene. After the mutant is operably ligated to a target gene, the expression efficiency of the target gene can be significantly improved, thereby providing an expression element having great application potential for the modification of a key gene in the synthesis pathway of a target compound. Where the mutant is applied to the production of a target compound, the conversion rate of the target compound can be significantly improved, providing a strong promoter having great potential for industrial fermentation of target compounds such as amino acids and organic acids.

In some embodiments, the polynucleotide having promoter activity provided by the present disclosure has 0.4 to 13 folds or more improved promoter activity as compared with the promoter activity of the wild-type *mdh* gene promoter.

In some embodiments, the present disclosure provides a transcription expression cassette, a recombinant expression vector, and a recombinant host cell, which comprise the aforesaid polynucleotide having promoter activity. In the transcription expression cassette, the recombinant expression vector and the recombinant host cell, the polynucleotide having promoter activity is operably ligated with a target gene, and efficient expression of a key gene in the synthesis pathway of a target compound can be realized.

In some embodiments, the present disclosure provides a method for preparing a protein capable of increasing the expression level of a protein related to the synthesis of amino acids, organic acids or gene expression regulatory proteins, thereby enabling efficient production of a target compound.

In some embodiments, the present disclosure provides a method for producing a target compound capable of improving the expression efficiency of a protein related to the synthesis of a target compound by using the aforesaid polynucleotide having promoter activity, and then the yield and conversion rate of the target compound can be effectively improved, and large-scale industrial production of the target compound can be realized.

### BRIEF DESCRIPTION OF THE DRAWINGS

FIG. 1 shows a plasmid profile of pEC-XK99E-P*_{mdh}*-rfp;
FIG. 2 shows a fluorescence result of mutant clones grown on a culture plate.

### DETAILED DESCRIPTION

When used in combination with the term "comprising" in the claims and/or the specification, the word "a" or "an" may refer to "one", or refer to "one or more", "at least one", and "one or more than one".

As used in the claims and specification, the term "comprise", "have/has", "include", or "contain" is intended to be inclusive or open-ended, and does not exclude additional or unquoted elements or method steps.

Throughout the present disclosure document, the term "about" means that a value includes the standard deviation of the error of a device or method used to measure the value.

It is applicable to the content disclosed herein that the term "or" is defined only as alternatives and "and/or", but the term "or" used herein refers to "and/or" unless otherwise expressly stated to be merely alternatives or mutual exclusion between alternatives.

The selected/optional/preferred "numerical range", when used in the claims or the specification, includes not only the numerical endpoints at both ends of the range, but also all natural numbers covered between the preceding numerical endpoints with respect to these numerical endpoints.

As used herein, the term "malate dehydrogenase" catalyzes the reversible conversion between malic acid and oxaloacetic acid and is coded by *mdh* gene. In some embodiments, the *mdh* gene in the present disclosure is derived from *Corynebacterium glutamicum.*

As used herein, the term "phosphoenolpyruvate carboxylase" (Phosphopyruvate carboxylase) catalyzes the conversion of phosphoenolpyruvate (PEP) to oxaloacetic acid and is coded by *ppc* gene.

As used herein, the term "pyruvate carboxylase" catalyzes the reversible carboxylation of pyruvic acid to form acetyl oxalate and is coded by *pyc* gene.

As used herein, the term "polynucleotide" refers to a polymer composed of nucleotides. Polynucleotide can be in the form of an individual fragment or a component of a larger nucleotide sequence structure, and it is derived from a nucleotide sequence separated at least once in quantity or concentration and can be recognized, manipulated and restored from the sequence and its component nucleotide sequences by standard molecular biology methods (such as using a cloning vector). When a nucleotide sequence is represented by a DNA sequence (i.e., A, T, G, C), this also includes an RNA sequence (i.e., A, U, G, C), wherein "U" replaces "T". In other words, "polynucleotide" refers to a nucleotide polymer that has been removed from other nucleotides (an individual fragment or an entire fragment), or may be a constituent part or component of a larger nucleotide structure, such as an expression vector or a polycistronic sequence. The polynucleotide includes DNA, RNA and cDNA sequences.

As used herein, the term "wild-type" refers to an object that can be found in nature. For example, a polypeptide or a polynucleotide sequence present in an organism that can be isolated from one source in nature and has not been intentionally modified by human in the laboratory is naturally occurring. As used herein, "naturally occurring" and "wild-type" are synonymous. In some embodiments, the wild-type promoter in the present disclosure refers to the wild-type *mdh* gene promoter, i.e., the polynucleotide having the sequence as shown in SEQ ID NO: 1.

As used herein, the term "mutant" refers to a polynucleotide or polypeptide that, relative to the "wild-type" or "comparative" polynucleotide or polypeptide, comprises alternation(s) (*i.e*., substitution, insertion and/or deletion of a polynucleotide) at one or more (*e.g*., several) positions, wherein the substitution refers to a substitution of a different nucleotide for a nucleotide that occupies one position; the deletion refers to removal of a nucleotide that occupies certain position; and the insertion refers to an addition of a nucleotide after the nucleotide adjacent to and immediately following the occupied position.

In some embodiments, the "mutation" in the present disclosure is "substitution", which is a mutation caused by a substitution of one base in the nucleotide with another different base, which is also referred to as a base substitution or a point mutation.

Specifically, the sequence as set forth in SEQ ID NO: 1 is the sequence of *mdh* gene promoter, which comprises a promoter core region having a nucleotide sequence of "CGTCAAGATCACCCAAAACTGGTGGCTGTTCTCTTTTAAGCGGGATAGCATGGGTT CTTA", wherein the underlined part is the sequence of the -10 region. The mutant in the present disclosure introduces mutated nucleotides in the -10 region and the 17 bp before the -10 region, and it is found that the promoter activity of the mutants after the introduction of mutations at the aforesaid positions is significantly enhanced, and a series of novel strong promoters are obtained, providing abundant expression regulatory elements for the efficient synthesis of target compounds.

In some embodiments, the polynucleotide having promoter activity refers to a mutant of the polynucleotide comprising the sequence as set forth in SEQ ID NO: 1, the mutant has mutated nucleotide(s) at one or more positions in position 105 to position 126 of the sequence as set forth in SEQ ID NO: 1 and does not comprise the polynucleotide mutating to TTCTCTTTTAAGCGGGATAGCA at position 105 to position 126 of the sequence as set forth in SEQ ID NO: 1. The mutant has improved promoter activity compared with the polynucleotide comprising the sequence as set forth in SEQ ID NO: 1.

In some embodiments, the mutant of the polynucleotide comprising the sequence as set forth in SEQ ID NO: 1 has 0.4 to 13 folds or more improved promoter activity as compared with the promoter activity of the polynucleotide comprising the sequence as set forth in SEQ ID NO: 1.

In some more specific embodiments, the mutant has 0.4, 1.7, 1.8, 2.0, 2.1, 2.3, 2.4, 3.1, 3.2, 3.4, 3.6, 4.0, 4.3, 4.5, 4.6, 4.7, 4.8, 4.9, 5.0, 5.1, 5.2, 5.3, 5.6, 5.7, 5.9, 6.1, 6.2, 6.4, 6.5, 6.6, 6.7, 6.8, 7.1, 7.3, 7.4, 7.5, 7.6, 7.7, 7.8, 8.2, 9.6, 13.0 folds improved promoter activity as compared with the promoter activity of the polynucleotide comprising the sequence as set forth in SEQ ID NO: 1.

As used herein, the term "promoter" refers to a nucleic acid molecule, which is typically located upstream of the coding sequence of the target gene, providing a recognition site for RNA polymerase, and is located upstream in the 5' direction of the transcription initiation site of mRNA. It is an untranslated nucleic acid sequence to which RNA polymerase binds and which initiates transcription of the target gene. In the synthesis of ribonucleic acid (RNA), the promoter can interact with transcription factors and regulate gene transcription and control the initiation time and degree of gene expression (transcription), and it comprises a core promoter region and regulatory region, functions like a "switch", determines the activity of the gene and then controls which kind of protein the cell starts to produce.

As used herein, the term "promoter core region" refers to a nucleic acid sequence located in the promoter region of prokaryotes, and it is the core sequence region that plays the role of promoter, mainly including the -35 region, the -10 region, and the region between the - 35 region and the -10 region, and the transcription initiation site, the -35 region being the recognition site of RNA polymerase, and the -10 region being the binding site of RNA polymerase. In some embodiments, the polynucleotide having promoter activity of the present disclosure is a mutant that comprises a promoter core region of *mdh* gene and introduces mutation in the -10 region and the 17 bp before the -10 region of the promoter core region, so as to obtain promoter activity significantly improved as compared with the promoter activity of the *mdh* gene promoter.

As used herein, the term "sequence identity" and "identity percentage" refer to the percentage of nucleotides or amino acids that are the same (i.e., identical) between two or more polynucleotides or polypeptides. The sequence identity between two or more polynucleotides or polypeptides can be determined by aligning the nucleotide or amino acid sequences of polynucleotides or polypeptides, scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides can be different at one position, for example, by containing a different nucleotide (i.e., substitution or mutation) or deletion of a nucleotide (i.e., nucleotide insertion or nucleotide deletion in one or two polynucleotides). Polypeptides can be different at one position, for example, by containing a different amino acid (i.e., substitution or mutation) or deletion of an amino acid (i.e., amino acid insertion or amino acid deletion in one or two polypeptides). The sequence identity can be calculated by dividing the number of positions at which nucleotide or amino acid residues are identical by the total number of nucleotide or amino acid residues in the polynucleotide or the polypeptide. For example, the identity percentage can be calculated by dividing the number of positions at which nucleotide or amino acid residues are identical by the total number of nucleotide or amino acid residues in the polynucleotide or polypeptide, and multiplying the result by 100.

In some embodiments, two or more sequences or subsequences, when compared and aligned at maximum correspondence by the sequence alignment algorithm or by the visual inspection measurement, have at least 80%, 81%, 82%, 83%, 84%, 85%, 86%, 87%, 88%, 89%, 90%, 91%, 92%, 93%, 94%, 95%, 96%, 97%, 98%, or 99% "sequence identity" or "percent identity" of nucleotides. In some embodiments, the sequence is substantially identical over the full length of either or both of the compared biopolymers (*e.g.*, polynucleotides).

As used herein, the term "complementary" refers to hybridization or base pairing between nucleotides or nucleotides, *e.g*., between two chains of a double-stranded DNA molecule or between an oligonucleotide primer and a primer binding site on a single-stranded nucleotide to be sequenced or amplified.

As used herein, the term "high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleaved and denatured salmon sperm DNA, and 50% formamide. Finally, the vector material is washed three times at 65°C with 2X SSC and 0.2% SDS, each for 15 min.

As used herein, the term "very high-stringent conditions" means that following the standard DNA blotting procedures, a probe of at least 100 nucleotides in length pre-hybridizes or hybridizes for 12 to 24 hours at 42°C in 5X SSPE (saline sodium phosphate EDTA), 0.3% SDS, 200 µg/ml of cleaved and denatured salmon sperm DNA, and 50% formamide. Finally, the vector material is washed three times at 70°C with 2X SSC and 0.2% SDS, each for 15 min.

In some embodiments, the polynucleotide having promoter activity of the present disclosure can be used to initiate the expression of protein-coding genes. In some other embodiments, the polynucleotide having promoter activity of the present disclosure can be used to initiate the expression of non-coding genes.

As used herein, the term "expression" includes any step involving RNA production and protein production, including but not limited to transcription, post-transcriptional modification, translation, post-translational modification and secretion.

As used herein, the term "transcription expression cassette" refers to a recombinant expression element comprising a polynucleotide having promoter activity. In some embodiments, a transcription regulatory element that regulates the target gene may comprise elements such as an enhancer, a silencer, an insulator in addition to the polynucleotide having promoter activity. In some embodiments, the target gene in the present disclosure is specifically a protein-coding gene. "Operably ligating" of a target gene to a polynucleotide having promoter activity means that a polynucleotide having promoter activity is functionally ligated to a target gene to initiate and mediate the transcription of the target gene, and the operably ligating can be in any of the ways described by those skilled in the art.

As used herein, the term "vector" refers to a DNA construct containing a DNA sequence operably ligated with suitable regulatory sequences, so as to express a target gene in a suitable host. "Recombinant expression vector" refers to a DNA structure used to express, for example, a polynucleotide coding a desired polypeptide. The recombinant expression vector may include, for example, i) a collection of genetic elements that have regulatory functions on gene expression, such as promoters and enhancers; ii) a structure or coding sequence transcribed into mRNA and translated into a protein; and iii) transcription subunits which appropriately transcribe and translate the start and stop sequences. The recombinant expression vector is constructed in any suitable way. The property of the vector is not critical, and any vector can be used, including plasmids, viruses, phages and transposons. Possible vectors for use in the present disclosure include, but are not limited to, chromosomal, non-chromosomal and synthetic DNA sequences, such as bacterial plasmids, phage DNA, yeast plasmids and vectors derived from combinations of plasmids and phage DNA, and DNA from viruses such as vaccinia virus, adenovirus, fowl pox virus, baculovirus, SV40 and pseudorabies virus, etc. In the present disclosure, "recombinant expression vector" and "recombinant vector" can be used interchangeably.

As used herein, the term "target RNA" includes functional RNAs that play a role in processes such as genetic coding, translation, regulation, gene expression, etc. In the present disclosure, the target RNA ligated to a polynucleotide having promoter activity can be any functional RNA in the art.

In some embodiments, the target RNA is tRNA or sRNA. In some other embodiments, the target RNA can also be other types of RNA such as sgRNA, crRNA, tracrRNA, miRNA, siRNA, etc.

As used herein, the term "target gene" relates to any of genes that are ligated to the polynucleotide having promoter activity of the present disclosure to regulate its transcriptional level.

In some embodiments, the target gene is a coding gene of a protein related to the synthesis of a target compound. In some embodiments, the target gene is a coding gene of a gene expression regulatory protein. In some embodiments, the target gene is a coding gene of a protein related to membrane transport.

Exemplarily, the target gene is a coding gene that codes an enzyme related to biosynthesis of a target compound, a coding gene of an enzyme related to the reducing power, a coding gene of an enzyme related to glycolysis or TCA cycle, or a coding gene of an enzyme related to the release of a target compound, etc.

Exemplarily, the target gene includes at least one of the following genes: pyruvate carboxylase gene, phosphoenolpyruvate carboxylase gene, γ-glutamyl kinase gene, glutamate semialdehyde dehydrogenase gene, pyrroline-5-carboxylate reductase gene, amino acid transport protein gene, ptsG system related gene, pyruvate dehydrogenase gene, homoserine dehydrogenase gene, oxaloacetic acid decarboxylase gene, gluconic acid repressor gene, glucose dehydrogenase gene, aspartate kinase gene, aspartate semialdehyde dehydrogenase gene, aspartate ammonia lyase gene, dihydrodipicolinate synthase gene, dihydropyridinic acid reductase gene, succinyl diaminopimelate aminotransferase gene, tetrahydrodipicolinate succinylase gene, succinyl diaminopimelate deacylase gene, diaminopimelic acid epimerase gene, diaminopimelic acid deacylase gene, glyceraldehyde-3-phosphate dehydrogenase gene, transketolase gene, diaminopimelate dehydrogenase gene, glyceraldehyde-3-phosphate dehydrogenase gene.

As used herein, the term "target compound" can be selected from amino acids, organic acids, and can also be selected from other types of compounds that can be obtained by biosynthesis in the art.

In some embodiments, the target compound is an "amino acid" or "L-amino acid". "Amino acid" or "L-amino acid" typically refers to refers to a basic constituent unit of a protein in which amino and carboxyl group are bounded to the same carbon atom. Exemplarily, the amino acid is selected from one or more of the following: glycine, alanine, valine, leucine, isoleucine, threonine, serine, cysteine, glutamine, methionine, aspartic acid, asparagine, glutamic acid, lysine, arginine, histidine, phenylalanine, tyrosine, tryptophan, proline, hydroxyproline, 5-aminolevulinic acid or derivatives of any of the above amino acids. In addition, the amino acid can also be other types of amino acids in the art.

In some embodiments, the target compound is an organic acid. The organic acid can be an acidic organic compound, e.g., those compounds that contain a carboxyl group and a sulfonic acid group. Exemplarily, the organic acid includes one or more of the following: lactic acid, acetic acid, succinic acid, butyric acid, palmitic acid, oxalic acid, oxaloacetic acid, tartaric acid, citric acid, propionic acid, hexenoic acid, decanoic acid acid, caprylic acid, valeric acid, malic acid or derivatives of any of the above organic acids. In addition, the organic acid can also be any other types of organic acids in the art.

The term "protein-coding gene" in the present disclosure refers to a DNA molecule capable of directing the synthesis of a protein by certain rules, and the process by which a protein-coding gene directing protein synthesis generally includes transcription process using double-stranded DNA as a template and translation using mRNA as a template. The protein-coding gene contains CDS sequence (Coding Sequence) directing the production of protein-coding mRNA.

Exemplarily, the protein-coding gene includes, but is not limited to, coding genes of proteins related to the synthesis of a target compound, and in some embodiments, the protein-coding gene relates to coding genes of proteins related to the synthesis of L-amino acid. Exemplarily, the proteins related to the synthesis of L-amino acid include, but are not limited to, one or a combination of two or more of: pyruvate carboxylase, phosphoenolpyruvate carboxylase, γ-glutamyl kinase, glutamate semialdehyde dehydrogenase, pyrroline-5-carboxylate reductase, amino acid transport protein, ptsG system, pyruvate dehydrogenase, homoserine dehydrogenase, oxaloacetic acid decarboxylase, gluconic acid repressor protein, glucose dehydrogenase. In some embodiments, the protein related to the synthesis of L-amino acid is a protein related to the synthesis of L-lysine, including one or a combination of two or more of: aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, lysine transport protein, transketolase, diaminopimelate dehydrogenase and pyruvate carboxylase.

In some embodiments, the protein-coding gene relates to a coding gene of a protein related to the synthesis of organic acids. Exemplarily, the protein-coding gene is used to code a protein related to the synthesis of oxaloacetic acid, a protein related to the synthesis of citric acid, or is used to code a protein related to the synthesis of succinic acid.

In some embodiments, the protein-coding gene relates to a coding gene of a related enzyme that promotes oxaloacetic acid synthesis. Exemplarily, the protein-coding gene is a coding gene *ppc* gene of phosphoenolpyruvate carboxylase. As reported in the existing literature^{[1]}, expression of the related enzyme that promotes oxaloacetic acid synthesis can increase the yield of 5-aminolevulinic acid.

In the present disclosure, the term "host cell" refers to any cell type that is easily transformed, transfected, transduced, etc., with a transcription initiation element or expression vector comprising the polynucleotide of the present disclosure. The term "recombinant host cell" covers a host cell that differs from the parent cell after introduction of a transcription initiation element or a recombinant expression vector, and the recombinant host cell is specifically realized by transformation.

In the present disclosure, the term "transformation" has the meaning generally understood by those skilled in the art, i.e., the process of introducing exogenous DNA into a host. The transformation method includes any method for introducing nucleic acid into a cell, including but not limited to electroporation, calcium phosphate (CaPO₄) precipitation, calcium chloride (CaCh) precipitation, microinjection, polyethylene glycol (PEG) method, DEAE-dextran method, cationic liposome method and lithium acetate-DMSO method.

The host cell of the present disclosure can be a prokaryotic cell or an eukaryotic cell, as long as it is a cell to which the polynucleotide having promoter activity of the present disclosure can be introduced. In one embodiment, the host cell refers to a prokaryotic cell. Specifically, the host cell is derived from a microorganism suitable for producing amino acids, organic acids by fermentation, such as genus *Corynebacterium,* genus *Brevibacterium,* genus *Arthrobacterium,* genus *Microbacterium* or genus *Escherichia.* Preferably, the host cell is *Corynebacterium glutamicum* from genus *Corynebacterium.* Among them, Corynebacterium glutamicum can be *Corynebacterium glutamicum* ATCC 13032, *Corynebacterium glutamicum* ATCC 13869, or *Corynebacterium glutamicum* ATCC 14067, and a mutant strain producing amino acids, especially lysine, or a derivative strain of *Corynebacterium glutamicum* prepared from the above strains. In some embodiments, the host cell in the present disclosure can be any type of strains having the capability for producing amino acids, including wild-type strains and recombinant strains.

Exemplarily, the host cell is a lysine-producing host cell. In some embodiments, the lysine-producing host cell can be a strain that expresses aspartate kinase that relieves feedback inhibition on the basis of *Corynebacterium glutamicum* ATCC 13032. Besides, the lysine-producing host cell can also be other types of strains that have the capability for producing lysine.

In some embodiments, the lysine-producing host cell may alsoinclude, but is not limited to, one or more genes selected from the following that are attenuated or reduced in expression:
a. *adhE* gene coding ethanol dehydrogenase;
b. *ackA* gene coding acetate kinase;
c. *pta* gene coding phosphate acetyltransferase;
d. *ldhA* gene coding lactate dehydrogenase;
e. *focA* gene coding formate transporter;
f. *pflB* gene coding pyruvate formate lyase;
g. *poxB* gene coding pyruvate oxidase;
h. *th*r*A* gene coding aspartate kinase I/ homoserine dehydrogenase I bifunctional enzyme;
i. *thrB* gene coding homoserine kinase;
j. *ldcC* gene coding lysine decarboxylase; and
h. *cadA* gene coding lysine decarboxylase.

In some embodiments, the lysine-producing host cell may also include, but is not limited to, one or more genes selected from the following that are enhanced or overexpressed:
a. *dapA* gene coding dihydrodipyridine synthase that relieves feedback inhibition of lysine;
b. *dapB* gene coding dihydrodipicolinate reductase;
c. *ddh* gene coding diaminopimelate dehydrogenase;
d. *dapD* coding tetrahydrodipicolinate succinylase and dapE coding succinyl diaminopimelate deacylase
e. *asd* gene coding aspartate-semialdehyde dehydrogenase;
f*. ppc* gene coding phosphoenolpyruvate carboxylase;
g. *pntAB* gene coding nicotinamide adenine dinucleotide transhydrogenase;
i. *lysE* gene coding lysine transport protein.

Exemplarily, the host cell is a threonine-producing host cell. In some embodiments, the threonine-producing host cell is a strain that expresses aspartate kinase *LysC* gene that relieves feedback inhibition on the basis of *Corynebacterium glutamicum* ATCC 13032. In some other embodiments, the threonine-producing host cell can also be other types of strains having threonine-producing capability.

In some embodiments, the threonine-producing host cell contains one or more genes selected from the following that are enhanced or overexpressed:
a. *thrABC* gene coding threonine operon;
b. *hom* gene coding homoserine dehydrogenase that relieves feedback inhibition;
c. *gap* gene coding glyceraldehyde-3-phosphate dehydrogenase;
d. *pyc* gene coding pyruvate carboxylase;
e. *mqo* gene coding malate: quinone oxidoreductase;
f. *tkt* gene coding transketolase;
g. *gnd* gene coding 6-phosphogluconate dehydrogenase;
h. *thrE* gene coding threonine exporter;
i. *eno* gene coding enolase

Exemplarily, the host cell is an isoleucine-producing host cell. In some embodiments, the isoleucine-producing host cell is a strain that produces L-isoleucine by substituting alanine for the amino acid at position 323 of the *ilv*A gene of L-threonine dehydratase. In some other embodiments, the isoleucine-producing host cell can also be other types of strains having isoleucine-producing capability.

Exemplarily, the host cell is an O-acetylhomoserine-producing host cell. In some embodiments, the O-acetylhomoserine-producing host cell is a strain that produces O-acetylhomoserine by inactivating O-acetylhomoserine (thiol)-lyase. In some other embodiments, the O-acetylhomoserine-producing host cell can also be other types of strains having O-acetylhomoserine production capability.

Exemplarily, the host cell is a methionine-producing host cell. In some embodiments, the methionine-producing host cell is a strain that produces methionine by inactivating transcription regulators of methionine and cysteine. In some other embodiments, the methionine-producing host cell can also be other types of strains having methionine-producing capability.

In the present disclosure, the culture of the host cell can be carried out according to the conventional methods in art, including but not limited to well-plate culture, shaking culture, batch culture, continuous culture and fed-batch culture, etc., and various culture conditions such as temperature, time and pH value of the culture medium can be appropriately adjusted according to the actual situations.

Unless otherwise defined or clearly indicated, all technical and scientific terms in the present disclosure have the same meanings as commonly understood by those of ordinary skill in the art to which the present disclosure pertains.

### Mutant of mdh gene promoter core region

The present disclosure uses the sequence of the *mdh* gene promoter core region and introduces mutation in the -10 region and the 17 bp before the -10 region of the *mdh* gene promoter, to obtain a mutant of *mdh* gene promoter core region that comprises a mutated -10 region.

For the polynucleotide having promoter activity in the present disclosure, by mutating the *mdh* gene promoter core region, specifically, by introducing mutations to (TTCTCTTTTAAGCGGGATAGCA) in the vicinity of the -10 region of the *mdh* gene promoter core region, the mutants in the present disclosure have significantly improved promoter activity as compared with the wild-type promoter that comprises a *mdh* gene promoter core region, and are a series of novel strong promoters; the mutants, when applied to the fermentation of a target compound, exhibit higher conversion rate and yield of the target compound as compared with the wild-type promoter.

In some embodiments, the polynucleotide having promoter activity in the present disclosure has mutated nucleotides at 1, 2, 3, 4, 5, 6, 7, 8, 9, 10, 11, 12, 13, 14, 15, 16, 17, 18, 19 or 20 positions in position 105 to position 126 of the sequence as set forth in SEQ ID NO: 1, and has improved promoter activity as compared with the wild-type *mdh* gene promoter having the sequence as set forth in SEQ ID NO: 1.

In some embodiments, the polynucleotide having promoter activity in the present disclosure further includes a polynucleotide that is reversely complementary to the nucleotide sequence of the mutant of the *mdh* gene promoter as set forth in SEQ ID NO: 1. Moreover, the polynucleotide has improved promoter activity as compared with the wild-type *mdh* gene promoter having the sequence as set forth in SEQ ID NO: 1.

In some embodiments, the polynucleotide having promoter activity in the present disclosure further includes a polynucleotide that is reversely complementary to the sequence that hybridizes with the nucleotide sequence of a mutant of the *mdh* gene promoter as set forth in SEQ ID NO: 1 under high stringent hybridization conditions or very high stringent hybridization conditions. Moreover, the polynucleotide has a nucleotide sequence that is not TTCTCTTTTAAGCGGGATAGCA at position 105 to position 126 of the sequence as set forth in SEQ ID NO: 1; and the polynucleotide has improved promoter activity as compared with the wild-type *mdh* gene promoter having the sequence as set forth in SEQ ID NO: 1.

In some embodiments, the polynucleotide having promoter activity in the present disclosure has at least 80%, 81%, 82%, 83%, 84%, 85%, 86%, 87%, 88%, 89%, 90%, 91%, 92%, 93%, 94%, 95%, 96%, 97%, 98%, 99% sequence identity (including all ranges and percentages between thesenumbers) compared with the aforesaid polynucleotide. Moreover, the polynucleotide has a nucleotide sequence that is not TTCTCTTTTAAGCGGGATAGCA at position 105 to position 126 of the sequence as set forth in SEQ ID NO: 1; and the polynucleotide has improved promoter activity as compared with the wild-type *mdh* gene promoter having the sequence as set forth in SEQ ID NO: 1.

In some specific embodiments, the nucleotide sequence of the mutant corresponding to position 105 to position 126 of the sequence as set forth in SEQ ID NO: 1 is selected from any one of the group consisting of the following (P_{*mdh*-1}) to (P*_{mdh}*-49):
(P*_{mdh}*-1) CAGGTAACAACTGCGAGTACGG; (P*_{mdh}*-2) AGTCTGAGGGATATAATTAAGA;
(P*_{mdh}-*3) GGTCGTGGAGGACAAGTTAAGA; (P*_{mdh}*-4) CTACTACCATCGGGTGCTAAGG;
(P*_{mdh}*-5) GATAGGTAGGCGTTAAGTAAAC; (P*_{mdh}*-6) GGCAATTCGCGGGGAGGTAGAC;
(P*_{mdh}*-7) TATTGGGCGCGGCATTATAGGA; (P*_{mdh}*-8) GGGAACTGCTTTGGTAGTATCA;
(P*_{mdh}*-9) GTTCAGGTTTGGGCCGCTATGC; (P*_{mdh}*-10) TGATCGGGGCGCTTTGGTACGG;
(P*_{mdh}-*11) TATGAAATAAATTGGAGTAGGC; (P*_{mdh}*-12) AACGGTATCTTTCTCCATATAA;
(P*_{mdh}*-13) AACGGAGCGTGTACGACTAGAA; (P*_{mdh}*-14) TGCGCGGGTAAACGCTGTAGAG;
(P*_{mdh}*-15) TTGATGTCGTAGCGTAGTATTA; (P*_{mdh}*-16) AGCTCGGACTTTGGTGGTAGGC;
(P*_{mdh}*-17) CCGGCGCATTATAACGCTACAA; (P*_{mdh}*-18) CGGGGGTGTAAGTCGAGTAGAC;
(P*_{mdh}*-19) ACGGGTAGGTATTTCGCTAGGA; (P*_{mdh}*-20) TAGTGTATGCTTTTACATAGAC;
(P*_{mdh}-*21) AGGCAGCATGTACATGATATCG; (P*_{mdh}*-22) TTGAGCGGAGTTAATAGTACAC;
(P*_{mdh}*-23) ATACACACAACTTTCGATACTA; (P*_{mdh}*-24) ATTCCAACCTTATGTTATACTG;
(P*_{mdh}*-25) GAGGTAGTCGTTTGGGGTAGGA; (P*_{mdh}*-26) ATTGCTCGCGTAAATGGTAAAC;
(P*_{mdh}*-27) TGTTATTATGGGAGATGTAAGT; (P*_{mdh}*-28) GAAAAATGTACGTGTGTTAAAA;
(P*_{mdh}*-29) AGTCCTCAGTGGAGTGGTAACA; (P*_{mdh}*-30) TTTCCGGTGTTTTCGCGTAGAG;
(P*_{mdh}*-31) GGCGTCGGTAGGTATGTTATTG; (P*_{mdh}*-32) ACGGCGATCAGAGTGTGTAAGA;
(P*_{mdh}*-33) CCGTGCGTGATATGGGGTAGTG; (P*_{mdh}*-34) GGGTTGTGAGTTATCGGTATAA;
(P*_{mdh}*-35) TGCTCCGATGATGGTGGTAAGA; (P*_{mdh}*-36) GTTCTCACGGGTCCGTGTATGA;
(P*_{mdh}*-37) TTTCGGAATGGATGGTGTACGC; (P*_{mdh}*-38) ATTATGTCTTCTTTTGGTAAAA;
(P*_{mdh}*-39) GATCATAGGTTCTGTGGTAGAT; (P*_{mdh}*-40) CATGGCCGATCATGATGTAATA;
(P*_{mdh}*-41) TAGTGGCGGTGTACGTGTATGA; (P*_{mdh}*-42) ATTTGTTACAGGTATGGTAGTT;
(P*_{mdh}*-43) ACGGAACTTATTTCTGGTAACG; (P*_{mdh}*-44) ACCGGGAGGACTAATGGTATGG;
(P*_{mdh}*-45) GCCACCCCTTTTTCTGCTATTA; (P*_{mdh}*-46) TTGCGCCGGAGTTGCGGTATAG;
(P*_{mdh}*-47) GTTTACGGGTGTTCATGTAGTA; (P*_{mdh}*-48) TTTGTCTCGTATCTGTGTAAGA;
(P*_{mdh}*-49) ATTTTGGGCACTTAGTGTATTG.

In some specific embodiments, the nucleotide sequence of the mutant is selected from the sequence as set forth in any one of SEQ ID NOs: 3 to 51.

In some embodiments, the polynucleotide having promoter activity in the present disclosure has 0.4 to 13 folds or more improved promoter activity as compared with the promoter activity of the polynucleotide having the sequence as set forth in SEQ ID NO: 1, and further has 0.4, 1.7, 1.8, 2.0, 2.1, 2.3, 2.4, 3.1, 3.2, 3.4, 3.6, 4.0, 4.3, 4.5, 4.6, 4.7, 4.8, 4.9, 5.0, 5.1, 5.2, 5.3, 5.6, 5.7, 5.9, 6.1, 6.2, 6.4, 6.5, 6.6, 6.7, 6.8, 7.1, 7.3, 7.4, 7.5, 7.6, 7.7, 7.8, 8.2, 9.6, 13.0 folds enhanced promoter activity as compared with the promoter activity of a polynucleotide comprising the sequence as set forth in SEQ ID NO: 1.

### Recombinant expression vector and recombinant host cell

In some embodiments, amplification is carried out in the present disclosure using ATCC13032 genome (*Corynebacterium glutamicum* ATCC 13032, Gene ID: 2830649) as a template, and *mdh*-1 and *mdh*-2 as primers to obtain a DNA fragment of the *mdh* gene promoter; the N-terminus 180-bp fragment is amplified using *gapN*180-1 and *gapN*180-2 as primers, and a synthetic N-terminus 180 bp of *gapN* gene derived from *Streptococcus mutans* as a template; pEC-XK99E plasmid skeleton is amplified using PEC-XK99E-*rfp* plasmid as a template, and pEC-1 and pEC-2 as primers; DNA fragments of red fluorescent protein gene comprising a linker peptide are amplified using RFP-1/2 as primers, and PEC-XK99E-*rfp* plasmid as a template. The aforesaid *mdh* gene promoter fragment, N-terminus 180 bp fragment of *gapN,* DNA fragments of red fluorescent protein gene comprising a linker peptide, and pEC-XK99E plasmid skeleton are recombinantly ligated, to obtain a recombinant expression vector pEC-XK99E-P*_{mdh}*-*rpf*.

In some embodiments, using pEC-XK99E-P*_{mdh}*-*rfp* as a template, a fragment comprising a mutation region is amplified using *mdh-*N1 primer and TK-1 primer, a plasmid skeleton fragment is amplified using *mdh*-N2 primer and TK-2 primer in the present disclosure, and the aforesaid two kinds of fragments are recombinantly ligated to obtain a library of *mdh* gene promoter mutants.

In some embodiments, *Corynebacterium glutamicum* ATCC13032 is transformed using a plasmid of the library of *mdh* gene promoter mutants and pEC-XK99E-P*_{mdh}*-*rfp*, respectively, to obtain a recombinant host cell in the present disclosure. Mutants having enhanced fluorescence intensity are screened after well-plate culture, to obtain *mdh* gene promoter mutants having improved promoter activity.

In some embodiments, amplification is carried out using a plasmid of the library of *mdh* gene promoter mutants as a template, and *mdh*-P1, *mdh*-P2 as primers to obtain fragments of the respective *mdh* gene promoter mutants in the present disclosure; *ppc* gene fragments are amplified using *Corynebacterium glutamicum* ATCC 13032 genome as a template, and *ppc-*1/ppc-2 as primers; a plasmid skeleton is amplified using pEC-XK99E plasmid as a template, and PEC-1/PEC-2 as primers. The respective promoter mutant fragments are recombinantly ligated to the *ppc* gene fragments and the plasmid skeleton, to obtain *mdh* gene promoter mutant plasmids.

In some specific embodiments, the *mdh* gene promoter mutant plasmids include any one of the following: pEC*-*P_{*mdh-*1}*-ppc*, pEC*-*P_{*mdh-*2}*-ppc*, pEC*-*P_{*mdh-*3}*-ppc*, pEC*-*P_{*mdh-*4}*-ppc*, pEC-P_{*mdh*-5}-*ppc,* pEC*-*P_{*mdh-*6}-*ppc*, pEC-P_{*mdh-*7}*-ppc*, pEC-P_{*mdh-*8}*-ppc*, pEC*-*P_{*mdh-*9}*-ppc*, pEC-P_{*mdh-*10}*-ppc*, pEC-P_{*mdh-*11}*-ppc*, pEC*-P*_{*mdh-*12}*-ppc*, pEC-P_{*mdh-*13}*-ppc*, pEC-P_{*mdh-*14}*-ppc*, pEC*-P*_{*mdh-*15}*-ppc*, pEC-P_{*mdh*-16}-*ppc,* pEC*-*P_{*mdh-*17}-*ppc*, pEC*-*P_{*mdh-*18}*-ppc*, pEC*-*P_{*mdh-*19}-*ppc*, pEC*-*P_{*mdh-*20}*-ppc*, pEC*-*P_{*mdh-*21}*-ppc*, pEC*-*P_{*mdh-*22}*-ppc*, pEC-P_{*mdh-*23}*-ppc*, pEC*-*P_{*mdh-*24}*-ppc*, pEC-P_{*mdh*-25}-*ppc*, pEC-P_{*mdh-*26}*-ppc*, pEC-P_{*mdh-*27}*-ppc*, pEC*-*P_{*mdh-*28}*-ppc*, pEC-P_{*mdh-*29}*-ppc*, pEC*-*P_{*mdh-*30}-*ppc*, pEC-P_{*mdh*-31}*-ppc*, pEC-P_{*mdh*-32}-*ppc,* pEC*-P*_{*mdh-*33}*-ppc*, pEC-P_{*mdh-*34}*-ppc*, pEC-P_{*mdh-*35}*-ppc*, pEC-P_{*mdh-*36}*-ppc*, pEC*-P*_{*mdh-*37}*-ppc*, pEC-P_{*mdh-*38}*-ppc*, pEC*-*P_{*mdh*-39}*-ppc*, pEC*-P*_{*mdh-*40}*-ppc*, pEC-P_{*mdh*-41}*-ppc*, pEC*-P*_{*mdh-*42}*-ppc*, pEC-P_{*mdh-*43}-*ppc*, pEC-P_{*mdh-*44}*-ppc*, pEC-P_{*mdh-*45}*-ppc*, pEC-P_{*mdh-*46}*-ppc*, pEC-P_{*mdh-*47}*-ppc*, pEC-P_{*mdh*-48}-*ppc,* pEC-P_{*mdh-*49}*-ppc*.

In some other embodiments, a required recombinant vector can also be constructed in the present disclosure using the promoter mutant as set forth in any one of (P_{*mdh*-1}) to (P_{*mdh*-49}), according to the specific cloning needs.

In some embodiments, a *Corynebacterium glutamicum* SCgL30 strain in the present disclosure, the threonine at position 311 of aspartate kinase (coded by lysC gene) on the *Corynebacterium glutamicum* ATCC 13032 genome is mutated into isoleucine, to construct a strain SCgL30 that has certain lysine synthesis capability.

In some specific embodiments, a recombinant expression vector as set forth in any one of pEC-P_{*mdh*-1}*-ppc* to pEC-*P*_{*mdh-*49}*-ppc* is transformed into a SCgL30 strain to obtain a recombinant host cell in the present disclosure, and exemplarily, a recombinant expression vector as set forth in pEC-P_{*mdh-*39}*-ppc* is transformed into a SCgL30 strain to obtain a recombinant host cell in the present disclosure. In some other specific embodiments, a recombinant vector comprising the promoter mutant as set forth in any one of (P_{*mdh*-1}) to (P_{*mdh*-49}) can also be transformed into a SCgL30 strain to obtain a recombinant host cell in the present disclosure.

### Production process of a target compound

(1) A polynucleotide having promoter activity is operably ligated to a coding gene of a protein related to the synthesis of the target compound or to a coding gene of a gene expression regulatory protein, to obtain a recombinant expression vector of the protein related to the synthesis of the target compound or the gene expression regulatory protein, and the recombinant expression vector is used to transform a host cell, to obtain a recombinant host cell.
(2) The recombinant host cell is subject to fermentation culture, and the target compound is collected from the recombinant host cell or the culture broth of the recombinant host cell to complete the production process of the target compound.

In the aforesaid production process, since the polynucleotide has improved promoter activity, in the recombinant host cell, the transcription activity of the coding gene of the protein related to the synthesis of the target compound or the gene expression regulatory protein is increased, and the expression level of the protein related to the synthesis of the target compound or the gene expression regulatory protein is increased, thereby the yield of target compound is significantly increased.

In some embodiments, the target compound is an amino acid, and the coding gene of a protein related to the synthesis of the target compound refers to a coding gene of a protein related to the synthesis of an amino acid. In some embodiments, the target compound is L-amino acid, and the coding gene of a protein related to the synthesis of an amino acid refers to a coding gene of a protein related to the synthesis of L-amino acid.

In some specific embodiments, the protein related to the synthesis of an amino acid is phosphoenolpyruvate carboxylase. Increasing the expression of *ppc* by using a polynucleotide having promoter activity can enhance the synthesis from phosphoenolpyruvate (PEP) to oxaloacetic acid, which in turn promotes the production of an oxaloacetic acid precursor supply-dependent target compound , including aspartic acid family amino acids (lysine, threonine, isoleucine, methionine), and glutamic acid family amino acids (glutamic acid, proline, hydroxyproline, arginine, glutamine), etc.

In some specific embodiments, the host cell is *Corynebacterium glutamicum,* an important strain for producing target compounds such as amino acids, organic acids, etc., and after modification of *Corynebacterium glutamicum* using a polynucleotide, a transcription expression cassette or a recombinant expression vector having a strong constitutive promoter activity, the expression level of the protein related to the synthesis of the target compound in *Corynebacterium glutamicum* is significantly increased, resulting in a much higher capacity for long-term fermentative accumulation of the target compound in *Corynebacterium glutamicum.*

In some specific embodiments, the host cell is *Corynebacterium glutamicum* modified as follows: threonine at position 311 of aspartate kinase (coded by *lysC* gene) on the *Corynebacterium glutamicum* ATCC 13032 genome is mutated into isoleucine.

In some specific embodiments, the culture conditions of recombinant host cells are as follows: the recombinant host cells are inoculated into TSB liquid medium and cultured, and the culture is inoculated as a seed into a 24-well plate of which each well contains fermentation medium, and cultured at 30°C for 18 hours at a rotation speed of the plate shaker of 800 rpm. L-lysine production yield is detected after the fermentation.

Ingredients of the fermentation medium for lysine are as follows: glucose, 80g/L; yeast powder, 1g/L; soybean peptone, 1g/L; NaCl, 1g/L; Ammonium sulfate, 1g/L; Urea, 10g/L; K₂HPO₄·3H₂O, 1g/L; MgSO₄·7H₂O, 0.45g/L; FeSO₄·7H₂O, 0.05g/L; biotin, 0.4mg/L; vitamin B1, 0.1mg/L; MOPS, 40g/L; initial pH7.2. 25 µg/mL Kanamycin is supplemented to the medium.

In some specific embodiments, target compounds can be recovered from recombinant host cells or culture broths of recombinant cells by methods commonly used in the art, including but not limited to filtration, anion exchange chromatography, crystallization and HPLC.

In the art, methods for manipulating microorganisms are known, as explained in publications such as Current Protocols in Molecular Biology (Online ISBN: 9780471142720, John Wiley and Sons, Inc.), Microbial Metabolic Engineering: Methods and Protocols (Qiong Cheng Ed.,Springer) and Systems Metabolic Engineering: Methods and Protocols (Hal S.Alper Ed., Springer).

### Examples

Other objects, features and advantages of the present disclosure will become apparent from the following detailed description. However, it should be understood that the detailed description and specific examples (although indicating specific embodiments of the present disclosure) are given for explanatory purpose only, because various variations and modifications within the spirit and scope of the present disclosure will become obvious to those skilled in the art upon reading the detailed description.

The experimental techniques and methods used in the examples are conventional technical methods unless otherwise specified. For example, the experimental methods for which specific conditions are not indicated in the following examples are usually in accordance with conventional conditions, such as those described in Molecular Cloning: Laboratory Manual (New York: Cold Spring Harbor Laboratory Press, 1989), by Sambrook et al., or those suggested by manufacturers. Unless otherwise specified, the materials and reagents used in the examples can be obtained through formal commercial channels.

### Example 1. Construction of intensity characterization plasmid of Corynebacterium glutamicum mdh gene promoter

In the present disclosure, the promoter of *Corynebacterium glutamicum* malate dehydrogenase *mdh* (malate dehydrogenase) gene was selected to carry out intensity characterization, and a specific region mutation was further introduced to construct a promoter library, to obtain promoter mutants having enhanced expression intensity. In bacteria, the expression regulatory sequence and the N-terminus coding region of a gene are the key regions that affect gene expression. In the present disclosure, the expression intensity of a target promoter was characterized based on fluorescence intensity, using a method of sequentially ligating a gene upstream promoter, the N-terminus 180 bp coding region of a *Streptococcus mutans-derived* NADP⁺-dependent glyceraldehyde-3-phosphate dehydrogenase *gapN* gene, a flexible linker peptide and a red fluorescent protein gene *rfp.*

The present example first constructed a characterization vector of the *Corynebacterium glutamicum mdh* gene promoter. Based on the skeleton of a pEC-XK99E plasmid, 60 amino acids at the N-terminus of *Streptococcus mutans gapN* gene, a linker peptide and a red fluorescent protein gene were expressed by an expression regulatory region comprising a promoter upstream of *mdh* gene. The specific construction was as follows:

### (1) Amplification of fragments of mdh gene promoter and N-terminus sequence

Amplification primers were designed according to the published genome sequence of *Corynebacterium glutamicum* ATCC13032 (Gene ID:2830649), the annotation information of *Corynebacterium glutamicum mdh* gene, and the synthetic N-terminus 180 bp of *Streptococcus mutans* derived *gapN* gene.

A *mdh* wild-type promoter (the sequence is as set forth in SEQ ID NO: 1) was amplified using *mdh*-1/2 as primers and ATCC13032 genome as the template; the N-terminus 180 bp fragment was amplified using *gapN*180*-*1/2 as primers and the synthetic N-terminus 180 bp of *Streptococcus mutans* derived *gapN* gene (the sequence is as set forth in SEQ ID NO: 2) as a template.

### (2) Amplification of plasmid skeleton and rfp fragment

By using pEC-XK99E-*rfp*^{[2]} plasmid reported in literatures as a template, pEC-XK99E plasmid skeleton was amplified using pEC-1/2 as primers, and the DNA fragment of the red fluorescent protein gene comprising a linker peptide (the DNA sequence is GGCGGTGGCTCTGGAGGTGGTGGGTCCGGCGGTGGCTCT) were amplified using RFP-1/2 as primers.

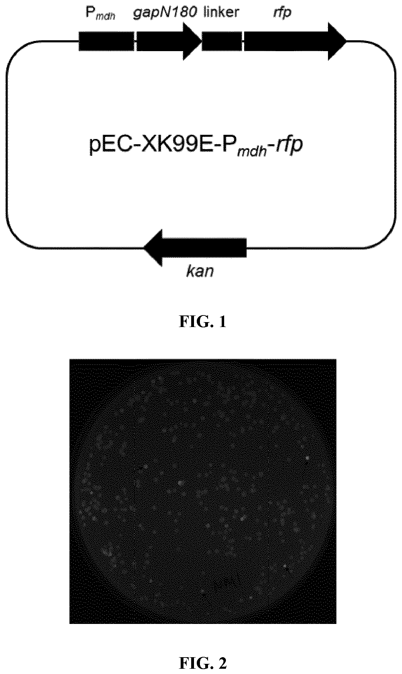

The above-obtained *mdh* gene promoter fragment, the N-terminus 180 bp fragment of *gapN,* the DNA fragment of red fluorescent protein gene comprising a linker peptide, and the plasmid skeleton were cloned and ligated via Vazyme's One Step Cloning Kit to obtain a pEC-XK99E-P*_{mdh}*-*rfp* characterization vector, of which the plasmid map is shown in FIG. 1. The sequences of the primers used above are shown in Table 1.

**Table 1**

| Primers | Nucleotide sequence | SEQ ID NO. |
|---|---|---|
| *mdh-*1 | CCTGATGCGGTATTTTCTCCCGATTCCCCATGATGCCCT | SEQ ID NO: 52 |
| | AAC | |
| *mdh-*2 | TAAACAATCCTCAATCCTTGTAGG | SEQ ID NO: 53 |
| *gapN180-1* | CAAGGATTGAGGATTGTTTAATGACAAAACAATATAAA AATTATGTC | SEQ ID NO: 54 |
| *gapN180-2* | CCACCTCCAGAGCCACCGCCTGAAAGTGATCGCCAAG CTGG | SEQ ID NO: 55 |
| pEC-1 | CTGCAGGCATGCAAGCTTGG | SEQ ID NO: 56 |
| pEC-2 | GGAGAAAATACCGCATCAGGC | SEQ ID NO: 57 |
| RFP-1 | GGCGGTGGCTCTGGAGGTGGTGGGTCCGGCGGTGGCT CTGCTTCCTCCGAAGACGTTATCAAAG | SEQ ID NO: 58 |
| RFP-2 | CCAAGCTTGCATGCCTGCAGTTAAGCACCGGTGGAGT GACGAC | SEQ ID NO: 59 |

### Example 2 Screening and intensity characterization of mutants of Corynebacterium glutamicum mdh gene promoter

### (1) Construction of mutant library of Corynebacterium glutamicum mdh gene promoter and preliminary screening

In the present disclosure, a series of mutations were introduced into the -10 region and the upstream 17 bp sequence of the *Corynebacterium glutamicum mdh* gene promoter, to construct a promoter library. The *mdh* promoter core region is as follows, wherein the underlined part is the main sequence of the -10 region of the promoter:

CGTCAAGATCACCCAAAACTGGTGGCTGTTCTCTTTAAGCGGATAGCAT
GGGTTCTTA;

The sequence in which mutations were introduced is as follows:

CGTCAAGATCACCCAAAACTGGTGGCTGNNNNNNNNNNNNNNNNNTANN
NTGGGTTCTTA.

By using the pEC-XK99E-P*_{mdh}*-*rfp* characterization vector constructed in Example 1 as a template, a fragment comprising the mutation region was amplified using *mdh*-N1 primer and TK-1 primer, and the plasmid skeleton fragment was amplified using *mdh*-N2 primer and TK-2 primer. The above two fragments were ligated via Vazyme's One Step Cloning Kit, and all clones obtained by gene promoter mutation were collected and plasmids were extracted to obtain a plasmid library of *mdh* gene promoter mutants.

*Corynebacterium glutamicum* ATCC13032 was transformed with the above plasmid library, and coated on a TSB solid plate supplemented with 25 µg/mL kanamycin. The plate, on which hundreds of clones were grown, was fluorescence photographed with a fluorescence imaging system, and mutants with improved expression intensity were preliminarily screened according to the fluorescence brightness of the clones. Ingredients (g/L) of the TSB plate medium are as follows: glucose, 5g/L; yeast powder, 5g/L; soy peptone, 9g/L; Urea, 3g/L; succinic acid, 0.5g/L; K₂HPO₄·3H₂O, 1g/L; MgSO4·7H₂O, 0.1g/L; biotin, 0.01mg/L; vitamin B1, 0.1mg/L; MOPS, 20g/L; agar powder, 15g/L. Meanwhile, *Corynebacterium glutamicum* ATCC13032 was transformed with the wild-type promoter characterization plasmid obtained in Example 1 to obtain a control strain. In the present disclosure, more than 10,000 clones of the promoter library were preliminarily screened to obtain mutants with enhanced fluorescence brightness. The screening plate is as shown in FIG. 2. The sequences of the primers used above are shown in Table 2.

**Table 2**

| Primers | Nucleotide sequence | SEQ ID NO. |
|---|---|---|
| *mdh-*N1 | TCACCCAAAACTGGTGGCTGNNNNNNNNNNNNNNNNTAN NNTGGGTTCTTAGAGGACCCCCTAC | SEQ ID NO: 60 |
| *mdh-*N2 | CAGCCACCAGTTTTGGGTGATC | SEQ ID NO: 61 |
| TK-1 | AACCTTCCATACGAACTTTGAAACG | SEQ ID NO: 62 |
| TK-2 | CAAAGTTCGTATGGAAGGTTCCG | SEQ ID NO: 63 |

### (2) Characterization and sequence analysis of the library of Corynebacterium glutamicum gene promoter mutants

### All mutants with enhanced fluorescence brightness shown by the fluorescence imaging in the above plate were cultured in a 96-well plate to characterize the intensity of promoters. Ingredients (g/L) of the TSB liquid medium are as follows: glucose, 5g/L; yeast powder, 5g/L; soy peptone, 9g/L; Urea, 3g/L; succinic acid, 0.5g/L; K₂HPO₄·3H₂O, 1g/L; MgSO₄·7H₂O, 0.1g/L; biotin, 0.01mg/L; vitamin B 1, 0.1mg/L; MOPS, 20g/L. 25 µg/mL Kanamycin was added to the culture medium. The clones with enhanced fluorescence brightness obtained in the plate and the wild-type promoter control strain were inoculated with toothpicks into a 96-well plate containing 200µl TSB liquid culture medium in each well, with three parallel strains for each strain. At a rotation speed of the plate shaker of 800rpm, after culturing at 30°C for 24 hours, the fluorescence intensities of the strains were detected. For the strains with improved fluorescence intensity as compared with the wild-type control, the mutation region fragments were amplified using pEC-F(TACGGTTCCTGGCCTTTTGC) and RFP-CX (CGGGTGTTTAACGTAAGCTTTG) primers and sequenced.

The characterization and sequencing results of the *mdh* promoter mutants are shown in Table 3. In the *mdh* gene promoter library, 49 promoter mutants (the nucleotide sequences of the corresponding mutant promoters were SEQ ID NOs: 3 to 51) with enhanced expression intensity as compared with the wild-type promoter were successfully obtained, and the improved folds ranged from 0.4 to 13.0 folds, indicating that mutations in the -10 region and the vicinity sequences of the *mdh* gene promoter could significantly enhance the promoter activity and provide plentiful elements for enhancing the expression of target genes.

**Table 3**

| Promoter No. | Fluorescence intensity (RFU/OD₆₀₀) | Multiple over the wild-type | Sequence of promoter mutation region (positions 105 to 127 of SEQ ID NO: 1) | Sequence No. of the complete promoter sequence |
|---|---|---|---|---|
| P*_{mdh}*-WT | 1662±54 | - | TTCTCTTTTAAGCGGGATAGCAT | SEQ ID NO: 1 |
| P*_{mdh}*-1 | 2362±83 | 0.4 | CAGGTAACAACTGCGAGTACGGT | SEQ ID NO: 3 |
| P*_{mdh}*-2 | 4469±170 | 1.7 | AGTCTGAGGGATATAATTAAGAT | SEQ ID NO: 4 |
| P*_{mdh}*-3 | 4543±84 | 1.7 | GGTCGTGGAGGACAAGTTAAGAT | SEQ ID NO: 5 |
| P*_{mdh}*-4 | 4734±511 | 1.8 | CTACTACCATCGGGTGCTAAGGT | SEQ ID NO: 6 |
| P*_{mdh}*-5 | 5023±60 | 2.0 | GATAGGTAGGCGTTAAGTAAACT | SEQ ID NO: 7 |
| P*_{mdh}*-6 | 5118±89 | 2.1 | GGCAATTCGCGGGGAGGTAGACT | SEQ ID NO: 8 |
| P*_{mdh}*-7 | 5491±262 | 2.3 | TATTGGGCGCGGCATTATAGGAT | SEQ ID NO: 9 |
| P*_{mdh}*-8 | 5617±117 | 2.4 | GGGAACTGCTTTGGTAGTATCAT | SEQ ID NO: 10 |
| P*_{mdh}*-9 | 6770±113 | 3.1 | GTTCAGGTTTGGGCCGCTATGCT | SEQ ID NO: 11 |
| P*_{mdh}*-10 | 6954±503 | 3.2 | TGATCGGGGCGCTTTGGTACGGT | SEQ ID NO: 12 |
| P*_{mdh}*-11 | 7319±213 | 3.4 | TATGAAATAAATTGGAGTAGGCT | SEQ ID NO: 13 |
| P*_{mdh}*-12 | 7616±194 | 3.6 | AACGGTATCTTTCTCCATATAAT | SEQ ID NO: 14 |
| P*_{mdh}-*13 | 8237±162 | 4.0 | AACGGAGCGTGTACGACTAGAAT | SEQ ID NO: 15 |
| P*_{mdh}*-14 | 8837±136 | 4.3 | TGCGCGGGTAAACGCTGTAGAGT | SEQ ID NO: 16 |
| P*_{mdh}*-15 | 9131±744 | 4.5 | TTGATGTCGTAGCGTAGTATTAT | SEQ ID NO: 17 |
| P*_{mdh}*-16 | 9288±128 | 4.6 | AGCTCGGACTTTGGTGGTAGGCT | SEQ ID NO: 18 |
| P*_{mdh}*-17 | 9393±119 | 4.7 | CCGGCGCATTATAACGCTACAAT | SEQ ID NO: 19 |
| P*_{mdh}*-18 | 9587±429 | 4.8 | CGGGGGTGTAAGTCGAGTAGACT | SEQ ID NO: 20 |
| P*_{mdh}*-19 | 9779±472 | 4.9 | ACGGGTAGGTATTTCGCTAGGAT | SEQ ID NO: 21 |
| P*_{mdh}*-20 | 10045±254 | 5.0 | TAGTGTATGCTTTTACATAGACT | SEQ ID NO: 22 |
| P*_{mdh}*-21 | 10137±194 | 5.1 | AGGCAGCATGTACATGATATCGT | SEQ ID NO: 23 |
| P*_{mdh}*-22 | 10266±263 | 5.2 | TTGAGCGGAGTTAATAGTACACT | SEQ ID NO: 24 |
| P*_{mdh}*-23 | 10341±63 | 5.2 | ATACACACAACTTTCGATACTAT | SEQ ID NO: 25 |
| P*_{mdh}*-24 | 10547±148 | 5.3 | ATTCCAACCTTATGTTATACTGT | SEQ ID NO: 26 |
| P*_{mdh}*-25 | 11031±608 | 5.6 | GAGGTAGTCGTTTGGGGTAGGAT | SEQ ID NO: 27 |
| P*_{mdh}*-26 | 11147±222 | 5.7 | ATTGCTCGCGTAAATGGTAAACT | SEQ ID NO: 28 |
| P*_{mdh}*-27 | 11440±452 | 5.9 | TGTTATTATGGGAGATGTAAGTT | SEQ ID NO: 29 |
| P*_{mdh}*-28 | 11464±901 | 5.9 | GAAAAATGTACGTGTGTTAAAAT | SEQ ID NO: 30 |
| P*_{mdh}*-29 | 11744±386 | 6.1 | AGTCCTCAGTGGAGTGGTAACAT | SEQ ID NO: 31 |
| P*_{mdh}*-30 | 11894±528 | 6.2 | TTTCCGGTGTTTTCGCGTAGAGT | SEQ ID NO: 32 |
| P*_{mdh}*-31 | 11948±287 | 6.2 | GGCGTCGGTAGGTATGTTATTGT | SEQ ID NO: 33 |
| P*_{mdh}*-32 | 12260±494 | 6.4 | ACGGCGATCAGAGTGTGTAAGAT | SEQ ID NO: 34 |
| P*_{mdh}*-33 | 12440±113 | 6.5 | CCGTGCGTGATATGGGGTAGTGT | SEQ ID NO: 35 |
| P*_{mdh}*-34 | 12582±872 | 6.6 | GGGTTGTGAGTTATCGGTATAAT | SEQ ID NO: 36 |
| P*_{mdh}*-35 | 12802±244 | 6.7 | TGCTCCGATGATGGTGGTAAGAT | SEQ ID NO: 37 |
| P*_{mdh}*-36 | 12833±384 | 6.7 | GTTCTCACGGGTCCGTGTATGAT | SEQ ID NO: 38 |
| P*_{mdh}*-37 | 12867±750 | 6.7 | TTTCGGAATGGATGGTGTACGCT | SEQ ID NO: 39 |
| P*_{mdh}*-38 | 13044±666 | 6.8 | ATTATGTCTTCTTTTGGTAAAAT | SEQ ID NO: 40 |
| P*_{mdh}*-39 | 13420±476 | 7.1 | GATCATAGGTTCTGTGGTAGATT | SEQ ID NO: 41 |
| P*_{mdh}*-40 | 13792±318 | 7.3 | CATGGCCGATCATGATGTAATAT | SEQ ID NO: 42 |
| P*_{mdh}*-41 | 13879±271 | 7.4 | TAGTGGCGGTGTACGTGTATGAT | SEQ ID NO: 43 |
| P*_{mdh}*-42 | 14143±452 | 7.5 | ATTTGTTACAGGTATGGTAGTTT | SEQ ID NO: 44 |
| P*_{mdh}*-43 | 14191±440 | 7.5 | ACGGAACTTATTTCTGGTAACGT | SEQ ID NO: 45 |
| P*_{mdh}*-44 | 14352±330 | 7.6 | ACCGGGAGGACTAATGGTATGGT | SEQ ID NO: 46 |
| P*_{mdh}*-45 | 14490±574 | 7.7 | GCCACCCCTTTTTCTGCTATTAT | SEQ ID NO: 47 |
| P*_{mdh}*-46 | 14704±537 | 7.8 | TTGCGCCGGAGTTGCGGTATAGT | SEQ ID NO: 48 |
| P*_{mdh}*-47 | 15265±125 | 8.2 | GTTTACGGGTGTTCATGTAGTAT | SEQ ID NO: 49 |
| P*_{mdh}*-48 | 17560±1126 | 9.6 | TTTGTCTCGTATCTGTGTAAGAT | SEQ ID NO: 50 |
| P*_{mdh}*-49 | 23336±967 | 13.0 | ATTTTGGGCACTTAGTGTATTGT | SEQ ID NO: 51 |

### Example 3 Application of Corynebacterium glutamicum mdh gene promoter mutants in L-lysine production

### (1) Construction of strains of Corynebacterium glutamicum mdh gene promoter mutants for the application in L-lysine production

In the present disclosure, T311I point mutation was first introduced into the aspartate kinase *lysC* gene of *Corynebacterium glutamicum* ATCC13032 strain, with the codon mutated from ACC to ATC, to obtain a SCgL30 strain. In the present disclosure, the *mdh* gene promoter mutant was further randomly applied to overexpress phosphonopyruvate carboxylase (PPC, NCBI-GeneID: 1019553, NCBI-Protein ID: NP_600799) gene to test its effect on L-lysine production.

The overexpression plasmid was constructed as follows: *ppc* gene was overexpressed using the P*_{mdh}*-39 promoter mutant on the basis of pEC-XK99E plasmid skeleton. A promoter mutant fragment was amplified using the corresponding promoter mutant plasmid screened in Example 2 as a template, and *mdh-*P1/*mdh-*P2 as primers; a *ppc* gene fragment was amplified using *Corynebacterium glutamicum* ATCC 13032 genome as a template, and *ppc-*1/*ppc-*2 as primers; a plasmid skeleton was amplified using pEC-XK99E plasmid as a template, and PEC-1/2 as primers. The above-obtained promoter mutant fragment, the *ppc* gene fragment and the plasmid skeleton fragment were cloned and ligated via Vazyme's One Step Cloning Kit to obtain a pEC-P_{*mdh*-39}-*ppc* plasmid. The pEC-XK99E control plasmid and the aforesaid plasmid were transformed into SCgL30 strains, respectively, to obtain a control strain SCgL 30 (pEC-XK99E) and a promoter mutant overexpression strain SCgL30(pEC-P*_{mdh-39}-ppc*)*.* The sequences of the primers used above are shown in Table 4.

**Table 4**

| Primers | Nucleotide sequence | SEQ ID NO. |
|---|---|---|
| *mdh-*P1 | CCTGATGCGGTATTTTCTCCCGATTCCCCATGATGCCCTAAC | SEQ ID NO: 64 |
| *mdh*-P2 | TCGCGTAAAAAATCAGTCATTAAACAATCCTCAATCCTTGTAGG | SEQ ID NO: 65 |
| *ppc*-1 | ATGACTGATTTTTTACGCGATGAC | SEQ ID NO: 66 |
| *ppc*-2 | CCAAGCTTGCATGCCTGCAGCTAGCCGGAGTTGCGCAGC | SEQ ID NO: 67 |
| PEC-1 | CTGCAGGCATGCAAGCTTGG | SEQ ID NO: 56 |
| PEC-2 | GGAGAAAATACCGCATCAGGC | SEQ ID NO: 57 |

### (2) Evaluation of L-lysine production capacity of a promoter mutant overexpressed strain

In order to test the effect of overexpression of *ppc* gene by P*_{mdh}*-39 promoter mutant in *Corynebacterium glutamicum* on L-lysine production by the strain, fermentation tests were carried out on SCgL30 (pEC-XK99E) and SCgL30 (pEC-P_{*mdh*-39}-*ppc*), respectively. Ingredients of the fermentation medium was as follows: glucose, 80g/L; yeast powder, 1g/L; soybean peptone, 1g/L; NaCl, 1g/L; ammonium sulfate, 1g/L; Urea, 10g/L; K₂UPO₄·3H₂O, 1g/L; MgSO₄·7H₂O, 0.45g/L; FeSO₄·7H₂O, 0.05g/L; biotin, 0.4mg/L; vitamin B1, 0.1mg/L; MOPS, 40g/L; initial pH7.2. 25 µg/mL Kanamycin was supplemented to the medium. Firstly, the strains were inoculated into TSB liquid medium to undergo culture for 8 hours, the cultures were inoculated as seeds into a 24-well plate containing 800 µl of fermentation medium in each well, and the inoculation amount was 12 µl. The initial OD₆₀₀ was controlled at about 0.1, and the culture was carried out at 30°C for 17 hours at a rotation speed of the plate shaker of 800rpm, with three parallel strains for each strain. After the fermentation, the L-lysine yield was detected by SBA biosensor and OD₆₀₀ was measured by a microplate reader. The results are shown in Table 5. The L-lysine yield using *mdh* promoter mutant overexpression strain SCgL30(pEC-P_{*mdh*-39}*-ppc*) was increased by 59%, indicating that the promoter mutants of the present disclosure can be used to enhance the expression of *ppc* gene and can be applied to L-lysine production.

**Table 5**

| Strains | OD₆₀₀ | L-lysine yield (g/L) |
|---|---|---|
| SCgL30(pEC-XK99E) | 17.7±1.2 | 4.50±0.0 |
| SCgL30(pEC-P_{*mdh-*39}*-ppc*) | 18.5±1.0 | 7.17±0.3 |

As shown by the above results, the promoter mutants of the present disclosure can be used to enhance the expression of PPC in *Corynebacterium glutamicum,* and thereby enhancing the synthesis from phosphoenolpyruvate (PEP) to oxaloacetic acid, thus can be applied to the production of an oxaloacetic acid precursor supply-dependent target product including aspartic acid family amino acids (lysine, threonine, isoleucine, methionine), glutamic acid family amino acids (glutamic acid, proline, hydroxyproline, arginine, glutamine), and to the biological production of amino acids such as 5-aminolevulinic acid for which oxaloacetic acid is an important metabolic precursor.

Since enhancing the expression and the activity of PPC can be used to increase the yield of a target compound such as 5-aminolevulinic acid^{[1]}, while all the promoter mutants of the present disclosure can be used to enhance the expression and the activity of PPC, thus the promoter mutants of the present disclosure can also be applied to the production of 5-aminolevulinic acid.

It has been verified that the promoter mutants of the present disclosure can improve the expression of a fusion protein of the N-terminus of *Streptococcus mutans* derived *gapN* gene and RFP, and can also be used to improve the expression of PPC, and the promoter mutants of the present disclosure can be also used to express other genes and be applied to the production of various products.
P*_{mdh}*-WT(SEQ ID NO: 1): 
cgattccccatgatgccctaacatcttgcaggtgaggggtacatattggggcaattcgggggtaattttgcagtatcgtcaag
atcacccaaaactggtggctgttctcttttaagcgggatagcatgggttcttagaggacccccctacaaggattgaggattgttta
*180bp coding region* (SEQ ID NO: 2) *of N-terminus of gapN* gene: 
atgacaaaacaatataaaaattatgtcaatggcgagtggaagctttcagaaaatgaaattaaaatctacgaaccggccagtg
gagctgaattgggttcagttccagcaatgagtactgaagaagtagattatgtttatgcttcagccaagaaagctcaaccagcttggcgatca
ctttca
P*_{mdh}*-1(SEQ ID NO: 3): 
cgattccccatgatgccctaacatcttgcaggtgaggggtacatattggggcaattcgggggtaattttgcagtatcgtcaag
atcacccaaaactggtggctgcaggtaacaactgcgagtacggtgggttcttagaggacccccctacaaggattgaggattgttta
P*_{mdh}*-2(SEQ ID NO: 4): 
cgattccccatgatgccctaacatcttgcaggtgaggggtacatattggggcaattcgggggtaattttgcagtatcgtcaag
atcacccaaaactggtggctgagtctgagggatataattaagatgggttcttagaggacccccctacaaggattgaggattgttta
P*_{mdh}*-3(SEQ ID NO: 5): 
cgattccccatgatgccctaacatcttgcaggtgaggggtacatattggggcaattcgggggtaattttgcagtatcgtcaag
atcacccaaaactggtggctgggtcgtggaggacaagttaagatgggttcttagaggacccccctacaaggattgaggattgttta
P*_{mdh}*-4(SEQ ID NO: 6): 
cgattccccatgatgccctaacatcttgcaggtgaggggtacatattggggcaattcgggggtaattttgcagtatcgtcaag
atcacccaaaactggtggctgctactaccatcgggtgctaaggtgggttcttagaggacccccctacaaggattgaggattgttta
P*_{mdh}*-5(SEQ ID NO: 7): 
cgattccccatgatgccctaacatcttgcaggtgaggggtacatattggggcaattcgggggtaattttgcagtatcgtcaag
atcacccaaaactggtggctggataggtaggcgttaagtaaactgggttcttagaggacccccctacaaggattgaggattgttta
P*_{mdh}*-6(SEQ ID NO: 8): 
cgattccccatgatgccctaacatcttgcaggtgaggggtacatattggggcaattcgggggtaattttgcagtatcgtcaag
atcacccaaaactggtggctgggcaattcgcggggaggtagactgggttcttagaggacccccctacaaggattgaggattgttta
P*_{mdh}*-7(SEQ ID NO: 9): 
cgattccccatgatgccctaacatcttgcaggtgaggggtacatattggggcaattcgggggtaattttgcagtatcgtcaag
atcacccaaaactggtggctgtattgggcgcggcattataggatgggttcttagaggacccccctacaaggattgaggattgttta
P*_{mdh}*-8(SEQ ID NO: 10): 
cgattccccatgatgccctaacatcttgcaggtgaggggtacatattggggcaattcgggggtaattttgcagtatcgtcaag
atcacccaaaactggtggctggggaactgctttggtagtatcatgggttcttagaggacccccctacaaggattgaggattgttta
P*_{mdh}*-9(SEQ ID NO: 11): 
cgattccccatgatgccctaacatcttgcaggtgaggggtacatattggggcaattcgggggtaattttgcagtatcgtcaag
atcacccaaaactggtggctggttcaggtttgggccgctatgctgggttcttagaggacccccctacaaggattgaggattgttta
P*_{mdh}*-10(SEQ ID NO: 12): 
cgattccccatgatgccctaacatcttgcaggtgaggggtacatattggggcaattcgggggtaattttgcagtatcgtcaag
atcacccaaaactggtggctgtgatcggggcgctttggtacggtgggttcttagaggacccccctacaaggattgaggattgttta
P*_{mdh}*-11(SEQ ID NO: 13): 
cgattccccatgatgccctaacatcttgcaggtgaggggtacatattggggcaattcgggggtaattttgcagtatcgtcaag
atcacccaaaactggtggctgtatgaaataaattggagtaggctgggttcttagaggacccccctacaaggattgaggattgttta
P*_{mdh}*-12(SEQ ID NO: 14): 
cgattccccatgatgccctaacatcttgcaggtgaggggtacatattggggcaattcgggggtaattttgcagtatcgtcaag
atcacccaaaactggtggctgaacggtatctttctccatataatgggttcttagaggacccccctacaaggattgaggattgttta
P*_{mdh}*-13(SEQ ID NO: 15): 
cgattccccatgatgccctaacatcttgcaggtgaggggtacatattggggcaattcgggggtaattttgcagtatcgtcaag
atcacccaaaactggtggctgaacggagcgtgtacgactagaatgggttcttagaggacccccctacaaggattgaggattgttta
P*_{mdh}*-14(SEQ ID NO: 16): 
cgattccccatgatgccctaacatcttgcaggtgaggggtacatattggggcaattcgggggtaattttgcagtatcgtcaag
atcacccaaaactggtggctgtgcgcgggtaaacgctgtagagtgggttcttagaggacccccctacaaggattgaggattgttta
P*_{mdh}*-15(SEQ ID NO: 17): 
cgattccccatgatgccctaacatcttgcaggtgaggggtacatattggggcaattcgggggtaattttgcagtatcgtcaag
atcacccaaaactggtggctgttgatgtcgtagcgtagtattatgggttcttagaggacccccctacaaggattgaggattgttta
P*_{mdh}*-16(SEQ ID NO: 18): cgattccccatgatgccctaacatcttgcaggtgaggggtacatattggggcaattcgggggtaattttgcagtatcgtcaag atcacccaaaactggtggctgagctcggactttggtggtaggctgggttcttagaggacccccctacaaggattgaggattgttta
P*_{mdh}*-17(SEQ ID NO: 19): 
cgattccccatgatgccctaacatcttgcaggtgaggggtacatattggggcaattcgggggtaattttgcagtatcgtcaag
atcacccaaaactggtggctgccggcgcattataacgctacaatgggttcttagaggacccccctacaaggattgaggattgttta
P*_{mdh}*-18(SEQ ID NO: 20): 
cgattccccatgatgccctaacatcttgcaggtgaggggtacatattggggcaattcgggggtaattttgcagtatcgtcaag
atcacccaaaactggtggctgcgggggtgtaagtcgagtagactgggttcttagaggacccccctacaaggattgaggattgttta
P*_{mdh}*-19(SEQ ID NO: 21): 
cgattccccatgatgccctaacatcttgcaggtgaggggtacatattggggcaattcgggggtaattttgcagtatcgtcaag
atcacccaaaactggtggctgacgggtaggtatttcgctaggatgggttcttagaggacccccctacaaggattgaggattgttta
P*_{mdh}*-20(SEQ ID NO: 22): 
cgattccccatgatgccctaacatcttgcaggtgaggggtacatattggggcaattcgggggtaattttgcagtatcgtcaag
atcacccaaaactggtggctgtagtgtatgcttttacatagactgggttcttagaggacccccctacaaggattgaggattgttta
P*_{mdh}*-21(SEQ ID NO: 23): 
cgattccccatgatgccctaacatcttgcaggtgaggggtacatattggggcaattcgggggtaattttgcagtatcgtcaag
atcacccaaaactggtggctgaggcagcatgtacatgatatcgtgggttcttagaggacccccctacaaggattgaggattgttta
P*_{mdh}*-22(SEQ ID NO: 24): 
cgattccccatgatgccctaacatcttgcaggtgaggggtacatattggggcaattcgggggtaattttgcagtatcgtcaag
atcacccaaaactggtggctgttgagcggagttaatagtacactgggttcttagaggacccccctacaaggattgaggattgttta
P*_{mdh}*-23(SEQ ID NO: 25): 
cgattccccatgatgccctaacatcttgcaggtgaggggtacatattggggcaattcgggggtaattttgcagtatcgtcaag
atcacccaaaactggtggctgatacacacaactttcgatactatgggttcttagaggacccccctacaaggattgaggattgttta
P*_{mdh}*-24(SEQ ID NO: 26): 
cgattccccatgatgccctaacatcttgcaggtgaggggtacatattggggcaattcgggggtaattttgcagtatcgtcaag
atcacccaaaactggtggctgattccaaccttatgttatactgtgggttcttagaggaccccctacaaggattgaggattgttta
P*_{mdh}*-25(SEQ ID NO: 27): 
cgattccccatgatgccctaacatcttgcaggtgaggggtacatattggggcaattcgggggtaattttgcagtatcgtcaag
atcacccaaaactggtggctggaggtagtcgtttggggtaggatgggttcttagaggacccccctacaaggattgaggattgttta
P*_{mdh}*-26(SEQ ID NO: 28): 
cgattccccatgatgccctaacatcttgcaggtgaggggtacatattggggcaattcgggggtaattttgcagtatcgtcaag
atcacccaaaactggtggctgattgctcgcgtaaatggtaaactgggttcttagaggacccccctacaaggattgaggattgttta
P*_{mdh}*-27(SEQ ID NO: 29): 
cgattccccatgatgccctaacatcttgcaggtgaggggtacatattggggcaattcgggggtaattttgcagtatcgtcaag
atcacccaaaactggtggctgtgttattatgggagatgtaagttgggttcttagaggacccccctacaaggattgaggattgttta
P*_{mdh}*-28(SEQ ID NO: 30): 
cgattccccatgatgccctaacatcttgcaggtgaggggtacatattggggcaattcgggggtaattttgcagtatcgtcaag
atcacccaaaactggtggctggaaaaatgtacgtgtgttaaaatgggttcttagaggacccccctacaaggattgaggattgttta
P*_{mdh}*-29(SEQ ID NO: 31): 
cgattccccatgatgccctaacatcttgcaggtgaggggtacatattggggcaattcgggggtaattttgcagtatcgtcaag
atcacccaaaactggtggctgagtcctcagtggagtggtaacatgggttcttagaggacccccctacaaggattgaggattgttta
P*_{mdh}*-30(SEQ ID NO: 32): 
cgattccccatgatgccctaacatcttgcaggtgaggggtacatattggggcaattcgggggtaattttgcagtatcgtcaag
atcacccaaaactggtggctgtttccggtgttttcgcgtagagtgggttcttagaggacccctacaaggattgaggattgttta
P*_{mdh}*-31(SEQ ID NO: 33): 
cgattccccatgatgccctaacatcttgcaggtgaggggtacatattggggcaattcgggggtaattttgcagtatcgtcaag
atcacccaaaactggtggctgggcgtcggtaggtatgttattgtgggttcttagaggacccccctacaaggattgaggattgttta
P*_{mdh}*-32(SEQ ID NO: 34): 
cgattccccatgatgccctaacatcttgcaggtgaggggtacatattggggcaattcgggggtaattttgcagtatcgtcaag
atcacccaaaactggtggctgacggcgatcagagtgtgtaagatgggttcttagaggacccccctacaaggattgaggattgttta
P*_{mdh}*-33(SEQ ID NO: 35): 
cgattccccatgatgccctaacatcttgcaggtgaggggtacatattggggcaattcgggggtaattttgcagtatcgtcaag
atcacccaaaactggtggctgccgtgcgtgatatggggtagtgtgggttcttagaggacccccctacaaggattgaggattgttta
P*_{mdh}*-34(SEQ ID NO: 36): 
cgattccccatgatgccctaacatcttgcaggtgaggggtacatattggggcaattcgggggtaattttgcagtatcgtcaag
atcacccaaaactggtggctggggttgtgagttatcggtataatgggttcttagaggacccccctacaaggattgaggattgttta
P*_{mdh}*-35(SEQ ID NO: 37): 
cgattccccatgatgccctaacatcttgcaggtgaggggtacatattggggcaattcgggggtaattttgcagtatcgtcaag
atcacccaaaactggtggctgtgctccgatgatggtggtaagatgggttcttagaggacccccctacaaggattgaggattgttta
P*_{mdh}*-36(SEQ ID NO: 38): 
cgattccccatgatgccctaacatcttgcaggtgaggggtacatattggggcaattcgggggtaattttgcagtatcgtcaag
atcacccaaaactggtggctggttctcacgggtccgtgtatgatgggttcttagaggacccccctacaaggattgaggattgttta
P*_{mdh}*-37(SEQ ID NO: 39): 
cgattccccatgatgccctaacatcttgcaggtgaggggtacatattggggcaattcgggggtaattttgcagtatcgtcaag
atcacccaaaactggtggctgtttcggaatggatggtgtacgctgggttcttagaggacccccctacaaggattgaggattgttta
P*_{mdh}*-38(SEQ ID NO: 40): 
cgattccccatgatgccctaacatcttgcaggtgaggggtacatattggggcaattcgggggtaattttgcagtatcgtcaag
atcacccaaaactggtggctgattatgtcttcttttggtaaaatgggttcttagaggacccccctacaaggattgaggattgttta
P*_{mdh}*-39(SEQ ID NO: 41): 
cgattccccatgatgccctaacatcttgcaggtgaggggtacatattggggcaattcgggggtaattttgcagtatcgtcaag
atcacccaaaactggtggctggatcataggttctgtggtagattgggttcttagaggacccccctacaaggattgaggattgttta
P*_{mdh}*-40(SEQ ID NO: 42): 
cgattccccatgatgccctaacatcttgcaggtgaggggtacatattggggcaattcgggggtaattttgcagtatcgtcaag
atcacccaaaactggtggctgcatggccgatcatgatgtaatatgggttcttagaggacccccctacaaggattgaggattgttta
P*_{mdh}*-41(SEQ ID NO: 43): 
cgattccccatgatgccctaacatcttgcaggtgaggggtacatattggggcaattcgggggtaattttgcagtatcgtcaag
atcacccaaaactggtggctgtagtggcggtgtacgtgtatgatgggttcttagaggacccccctacaaggattgaggattgttta
P*_{mdh}*-42(SEQ ID NO: 44): 
cgattccccatgatgccctaacatcttgcaggtgaggggtacatattggggcaattcgggggtaattttgcagtatcgtcaag
atcacccaaaactggtggctgatttgttacaggtatggtagtttgggttcttagaggacccccctacaaggattgaggattgttta
P*_{mdh}*-43(SEQ ID NO: 45): 
cgattccccatgatgccctaacatcttgcaggtgaggggtacatattggggcaattcgggggtaattttgcagtatcgtcaag
atcacccaaaactggtggctgacggaacttatttctggtaacgtgggttcttagaggacccccctacaaggattgaggattgttta
P*_{mdh}*-44(SEQ ID NO: 46): 
cgattccccatgatgccctaacatcttgcaggtgaggggtacatattggggcaattcgggggtaattttgcagtatcgtcaag
atcacccaaaactggtggctgaccgggaggactaatggtatggtgggttcttagaggacccccctacaaggattgaggattgttta
P*_{mdh}*-45(SEQ ID NO: 47): 
cgattccccatgatgccctaacatcttgcaggtgaggggtacatattggggcaattcgggggtaattttgcagtatcgtcaag
atcacccaaaactggtggctggccacccctttttctgctattatgggttcttagaggacccccctacaaggattgaggattgttta
P*_{mdh}*-46(SEQ ID NO: 48): 
cgattccccatgatgccctaacatcttgcaggtgaggggtacatattggggcaattcgggggtaattttgcagtatcgtcaag
atcacccaaaactggtggctgttgcgccggagttgcggtatagtgggttcttagaggacccccctacaaggattgaggattgttta
P*_{mdh}*-47(SEQ ID NO: 49): 
cgattccccatgatgccctaacatcttgcaggtgaggggtacatattggggcaattcgggggtaattttgcagtatcgtcaag
atcacccaaaactggtggctggtttacgggtgttcatgtagtatgggttcttagaggacccccctacaaggattgaggattgttta
P*_{mdh}*-48(SEQ ID NO: 50): 
cgattccccatgatgccctaacatcttgcaggtgaggggtacatattggggcaattcgggggtaattttgcagtatcgtcaag
atcacccaaaactggtggctgtttgtctcgtatctgtgtaagatgggttcttagaggacccccctacaaggattgaggattgttta
P*_{mdh}*-49(SEQ ID NO: 51):

cgattccccatgatgccctaacatcttgcaggtgaggggtacatattggggcaattcgggggtaattttgcagtatcgtcaag
atcacccaaaactggtggctgattttgggcacttagtgtattgtgggttcttagaggacccccctacaaggattgaggattgttta

### References:

[1] CN103981203A
[2] Wang, YC et al., Screening efficient constitutive promoters in Corynebacterium glutamicum based on time-series transcriptome analysis. Chinese Journal of Biotechnology, 2018, 34(11):1760 - 1771

All technical features disclosed in this specification can be combined in any way of combination. Each feature disclosed in this specification can also be replaced by another feature having the same, equal or similar function. Therefore, unless otherwise specified, each feature disclosed herein is merely an example of a series of equal or similar features.

In addition, according to the above description of the present disclosure, those skilled in the art can easily understand the key features of the present disclosure, and many modifications can be made to the invention to adapt to various use purposes and conditions without departing from the spirit and scope of the present disclosure, and therefore such modifications are also intended to fall within the scope of the appended claims.

## Claims

1. A polynucleotide having promoter activity, wherein the polynucleotide is selected from the group shown in any one of the following (i)-(iv):
(i) a mutant of a polynucleotide comprising the sequence as set forth in SEQ ID NO: 1, comprising a mutated nucleotide at one or more positions in position 105 to position 126 of the sequence as set forth in SEQ ID NO: 1;
(ii) a polynucleotide comprising a sequence complementary to the nucleotide sequence as set forth in (i);
(iii) a polynucleotide comprising a reversely complementary sequence of a sequence capable of hybridizing with the nucleotide sequence as set forth in (i) or (ii) under high stringent hybridization conditions or very high stringent hybridization conditions;
(iv) a polynucleotide having at least 90%, optionally at least 95%, preferably at least 97%, more preferably at least 98%, and most preferably at least 99% sequence identity compared with the nucleotide sequence as set forth in (i) or (ii);
wherein the polynucleotide as set forth in any one of (i) to (iv) has a nucleotide sequence that is not TTCTCTTTTAAGCGGGATAGCA at position 105 to position 126 of the sequence as set forth in SEQ ID NO: 1; and the polynucleotide as set forth in any one of (i) to (iv) has enhanced promoter activity compared with the polynucleotide having the sequence as set forth in SEQ ID NO: 1.

2. The polynucleotide having promoter activity according to claim 1, wherein the mutant has 0.4 to 13 folds or more improved promoter activity as compared with the promoter activity of the polynucleotide having the sequence as set forth in SEQ ID NO: 1.

3. The polynucleotide having promoter activity according to claim 1 or 2, wherein the nucleotide sequence of the mutant corresponding to position 105 to position 126 of the sequence as set forth in SEQ ID NO: 1 is selected from any one of the group consisting of the following (P*_{mdh}*-1) to (P*_{mdh}*-49):
(P*_{mdh}*-1) CAGGTAACAACTGCGAGTACGG;
(P*_{mdh}*-2) AGTCTGAGGGATATAATTAAGA;
(P*_{mdh}-*3) GGTCGTGGAGGACAAGTTAAGA;
(P*_{mdh}*-4) CTACTACCATCGGGTGCTAAGG;
(P*_{mdh}*-5) GATAGGTAGGCGTTAAGTAAAC;
(P*_{mdh}*-6) GGCAATTCGCGGGGAGGTAGAC;
(P*_{mdh}*-7) TATTGGGCGCGGCATTATAGGA;
(P*_{mdh}*-8) GGGAACTGCTTTGGTAGTATCA;
(P*_{mdh}*-9) GTTCAGGTTTGGGCCGCTATGC;
(P*_{mdh}*-10) TGATCGGGGCGCTTTGGTACGG;
(P*_{mdh}*-11) TATGAAATAAATTGGAGTAGGC;
(P*_{mdh}*-12) AACGGTATCTTTCTCCATATAA;
(P*_{mdh}*-13) AACGGAGCGTGTACGACTAGAA;
(*P_{mdh}*-14) TGCGCGGGTAAACGCTGTAGAG;
(P*_{mdh}*-15) TTGATGTCGTAGCGTAGTATTA;
(P*_{mdh}*-16) AGCTCGGACTTTGGTGGTAGGC;
(P*_{mdh}*-17) CCGGCGCATTATAACGCTACAA;
(P*_{mdh}*-18) CGGGGGTGTAAGTCGAGTAGAC;
(P*_{mdh}*-19) ACGGGTAGGTATTTCGCTAGGA;
(P*_{mdh}*-20) TAGTGTATGCTTTTACATAGAC;
(P*_{mdh}-*21) AGGCAGCATGTACATGATATCG;
(P*_{mdh}*-22) TTGAGCGGAGTTAATAGTACAC;
(P*_{mdh}*-23) ATACACACAACTTTCGATACTA;
(P*_{mdh}*-24) ATTCCAACCTTATGTTATACTG;
(P*_{mdh}*-25) GAGGTAGTCGTTTGGGGTAGGA;
(P*_{mdh}*-26) ATTGCTCGCGTAAATGGTAAAC;
(P*_{mdh}*-27) TGTTATTATGGGAGATGTAAGT;
(P*_{mdh}*-28) GAAAAATGTACGTGTGTTAAAA;
(P*_{mdh}*-29) AGTCCTCAGTGGAGTGGTAACA;
(P*_{mdh}*-30) TTTCCGGTGTTTTCGCGTAGAG;
(P*_{mdh}*-31) GGCGTCGGTAGGTATGTTATTG;
(P*_{mdh}*-32) ACGGCGATCAGAGTGTGTAAGA;
(P*_{mdh}*-33) CCGTGCGTGATATGGGGTAGTG;
(P*_{mdh}*-34) GGGTTGTGAGTTATCGGTATAA;
(P*_{mdh}*-35) TGCTCCGATGATGGTGGTAAGA;
(P*_{mdh}*-36) GTTCTCACGGGTCCGTGTATGA;
(P*_{mdh}*-37) TTTCGGAATGGATGGTGTACGC;
(P*_{mdh}*-38) ATTATGTCTTCTTTTGGTAAAA;
(P*_{mdh}*-39) GATCATAGGTTCTGTGGTAGAT;
(P*_{mdh}*-40) CATGGCCGATCATGATGTAATA;
(P*_{mdh}*-41) TAGTGGCGGTGTACGTGTATGA;
(P*_{mdh}*-42) ATTTGTTACAGGTATGGTAGTT;
(P*_{mdh}*-43) ACGGAACTTATTTCTGGTAACG;
(P*_{mdh}*-44) ACCGGGAGGACTAATGGTATGG;
(P*_{mdh}*-45) GCCACCCCTTTTTCTGCTATTA;
(P*_{mdh}*-46) TTGCGCCGGAGTTGCGGTATAG;
(P*_{mdh}*-47) GTTTACGGGTGTTCATGTAGTA;
(P*_{mdh}*-48) TTTGTCTCGTATCTGTGTAAGA;
(P*_{mdh}*-49) ATTTTGGGCACTTAGTGTATTG.

4. The polynucleotide having promoter activity according to any one of claims 1 to 3, wherein the nucleotide sequence of the mutant is selected from sequences as set forth in any one of SEQ ID NOs: 3 to 51.

5. A transcription expression cassette, comprising the polynucleotide having promoter activity according to any one of claims 1 to 4; optionally, the transcription expression cassette further contains a target gene that is operably ligated to the polynucleotide having promoter activity; preferably, the target gene is a protein coding gene.

6. A recombinant expression vector comprising the polynucleotide having promoter activity according to any one of claims 1 to 4, or the transcription expression cassette according to claim 5.

7. A recombinant host cell comprising the transcription expression cassette according to claim 5, or the recombinant expression vector according to claim 6.

8. The recombinant host cell according to claim 7, wherein the host cell is from genus *Corynebacterium,* genus *Brevibacterium,* genus *Arthrobacterium,* genus *Microbacterium* or genus *Escherichia*; preferably, the host cell is *Corynebacterium glutamicum* or *Escherichia coli*; more preferably, the host cell is *Corynebacterium glutamicum* ATCC 13032, *Corynebacterium glutamicum* ATCC 13869, *Corynebacterium glutamicum* ATCC 14067 or derivative strains of *Corynebacterium glutamicum.*

9. Use of the polynucleotide having promoter activity according to any one of claims 1 to 4, the transcription expression cassette according to claim 5, the recombinant expression vector according to claim 6, and the recombinant host cell according to claim 7 or 8 in at least one of the following:
a) enhancing the transcription level of a gene, or preparing a reagent or kit for enhancing the transcription level of a gene;
b) preparing a protein, or preparing a reagent or kit for preparing a protein;
c) producing a target compound, or preparing a reagent or kit for producing a target compound.

10. The use according to claim 9, wherein the protein is selected from a gene expression regulatory protein, a protein related to the synthesis of a target compound, or a protein related to membrane transport.

11. The use according to claim 9 or 10, wherein the target compound includes at least one of amino acids, organic acids;
optionally, the amino acids include one or a combination of two or more of the following: proline, hydroxyproline, lysine, glutamic acid, threonine, glycine, alanine, valine, leucine, isoleucine, serine, cysteine, glutamine, methionine, aspartic acid, asparagine, arginine, histidine, phenylalanine, tyrosine, tryptophan, 5-aminolevulinic acid or derivatives of any one of the above amino acids;
optionally, the organic acids include one or a combination of two or more of the following: citric acid, succinic acid, lactic acid, acetic acid, butyric acid, palmitic acid, oxalic acid, oxaloacetic acid, tartaric acid, propionic acid, hexenoic acid, decanoic acid, caprylic acid, valeric acid, malic acid or derivatives of any one of the above amino acids.

12. A construction method of a promoter mutant, including the following steps:
mutation step: mutating a polynucleotide having the sequence as set forth in SEQ ID NO: 1 so that there are mutated nucleotides at one or more positions in position 105 to position 126 of the sequence as set forth in SEQ ID NO: 1;
screening step: screening for a mutant of a polynucleotide having improved promoter activity as compared with the polynucleotide having the sequence as set forth in SEQ ID NO: 1, to obtain a promoter mutant.

13. The construction method according to claim 12, wherein the mutation step includes:
mutating the polynucleotide having the sequence as set forth in SEQ ID NO: 1 so that the nucleotides at position 105 to position 126 of the sequence as set forth in SEQ ID NO: 1 are mutated to a nucleotide sequence as set forth below: NNNNNNNNNNNNNNNNNTANNNT; wherein N is selected from A, T, C or G;
preferably, the promoter mutant has 0.4 to 13 folds or more improved promoter activity as compared with the promoter activity of the polynucleotide having the sequence as set forth in SEQ ID NO: 1.

14. A method for regulating transcription, wherein the method includes a step of operably ligating the polynucleotide having promoter activity according to any one of claims 1 to 4 to a target RNA or a target gene; optionally, the target RNA includes at least one of tRNA, sRNA, the target gene includes at least one of a coding gene of a protein related to the synthesis of a target compound, a coding gene of a gene expression regulatory protein and a coding gene of a protein related to membrane transport;
optionally, the target gene includes at least one of the following genes: pyruvate carboxylase gene, phosphoenolpyruvate carboxylase gene, γ-glutamyl kinase gene, glutamate semialdehyde dehydrogenase gene, pyrroline-5-carboxylate reductase gene, amino acid transport protein gene, ptsG system related gene, pyruvate dehydrogenase gene, homoserine dehydrogenase gene, oxaloacetic acid decarboxylase gene, gluconic acid repressor gene, glucose dehydrogenase gene, aspartate kinase gene, aspartate semialdehyde dehydrogenase gene, aspartate ammonia lyase gene, dihydrodipicolinate synthase gene, dihydropyridinic acid reductase gene, succinyl diaminopimelate aminotransferase gene, tetrahydrodipicolinate succinylase gene, succinyl diaminopimelate deacylase gene, diaminopimelic acid epimerase gene, diaminopimelic acid deacylase gene, glyceraldehyde-3-phosphate dehydrogenase gene, transketolase gene, diaminopimelate dehydrogenase gene.

15. A method for preparing a protein, wherein the method includes a step of expressing the protein by using the transcription expression cassette according to claim 5, the recombinant expression vector according to claim 6, or the recombinant host cell according to any one of claims 7 and 8; optionally, the protein is a protein related to the synthesis of a target compound, or a gene expression regulatory protein;
optionally, the method further includes a step of isolating or purifying the protein.

16. A method for producing a target compound, wherein the method includes a step of expressing a protein related to the synthesis of a target compound, a protein related to membrane transport, or a gene expression regulatory protein by using the transcription expression cassette according to claim 5, the recombinant expression vector according to claim 6, or the recombinant host cell according to any one of claims 7 and 8, and producing the target compound in the presence of the protein associated with the synthesis of the target compound, a protein related to membrane transport, or the gene expression regulatory protein;
optionally, the target compound includes at least one of amino acids, organic acids;
optionally, the amino acids include one or a combination of two or more of the following: lysine, glutamic acid, threonine, proline, hydroxyproline, glycine, alanine, valine, leucine, isoleucine, serine, cysteine, glutamine, methionine, aspartic acid, asparagine, arginine, histidine, phenylalanine, tyrosine, tryptophan, 5-aminolevulinic acid or derivatives of any one of the above amino acids;
optionally, the organic acids include one or a combination of two or more of the following: citric acid, succinic acid, lactic acid, acetic acid, butyric acid, palmitic acid, oxalic acid, oxaloacetic acid, tartaric acid, propionic acid, hexenoic acid, decanoic acid, caprylic acid, valeric acid, malic acid or derivatives of any one of the above organic acids;
optionally, the protein related to the synthesis of the target compound is a protein related to the synthesis of an L-amino acid; optionally, the protein related to the synthesis of an L-amino acid includes one or a combination of two or more of: pyruvate carboxylase, phosphoenolpyruvate carboxylase, γ-glutamyl kinase, glutamate semialdehyde dehydrogenase, pyrroline-5-carboxylate reductase, amino acid transport protein, ptsG system, pyruvate dehydrogenase, homoserine dehydrogenase, oxaloacetic acid decarboxylase, gluconic acid repressor, glucose dehydrogenase, aspartate kinase, aspartate semialdehyde dehydrogenase, aspartate ammonia lyase, dihydrodipicolinate synthase, dihydropyridinic acid reductase, succinyl diaminopimelate aminotransferase, tetrahydrodipicolinate succinylase, succinyl diaminopimelate deacylase, diaminopimelic acid epimerase, diaminopimelic acid deacylase, glyceraldehyde-3-phosphate dehydrogenase, transketolase, diaminopimelate dehydrogenase;
optionally, the method further includes a step of isolating or purifying the target compound.
